# EUROPÄISCHE PATENTSCHRIFT

(11) **EP 1 966 147 B1**
(45) Veröffentlichungstag und Bekanntmachung des Hinweises auf die Patenterteilung: **08.08.2012**
(21) Anmeldenummer: 06829299.4
(22) Anmeldetag: 05.12.2006
(51) Int. Cl.: A01N 43/56, A01N 47/48, A01N 53/00, C07D 231/14, C07D 401/12, C07D 409/12, C07D 213/61, C07D 333/28, C07D 231/12

(54) **CARBOXAMIDE ZUR BEKÄMPFUNG VON MIKROORGANISMEN IM PFLANZEN- UND MATERIALSCHUTZ**
CARBOXAMIDES FOR CONTROLLING MICRO-ORGANISMS IN PLANT AND MATERIAL PROTECTION
CARBOXAMIDES POUR LUTTER CONTRE LES MICRO-ORGANISMES DANS LA PROTECTION DES PLANTES ET DES MATERIAUX

(30) Priorität: 17.12.2005 DE 102005060466
(43) Veröffentlichungstag der Anmeldung: 10.09.2008
(73) Patentinhaber: Bayer CropScience AG, 40789 Monheim (DE)
(72) Erfinder: DUNKEL, Ralf, F-69001 Lyon (FR); ELBE, Hans-Ludwig, 42329 Wuppertal (DE); GREUL, Jörg, Nico, 42799 Leichlingen (DE); GAYER, Herbert, 40789 Monheim (DE); KRÜGER, Bernd-Wieland, 51467 Bergisch Gladbach (DE); VOERSTE, Arnd, 50674 Köln (DE); WACHENDORFF-NEUMANN, Ulrike, 56566 Neuwied (DE); DAHMEN, Peter, 41470 Neuss (DE)
(86) Internationale Anmeldenummer: PCT/EP2006/011653
(87) Internationale Veröffentlichungsnummer: WO 2007/068377

(56) Entgegenhaltungen:
- WO-A-02/38542
- WO-A-03/070705
- WO-A1-93/11117
- JOHN S. DAVIDSON: SYNTHESIS, 1979, Seiten 359-361, XP002421834
- WOLFGANG WALTER, KLAUS WOHLERS: LIEBIGS ANNALEN DER CHEMIE, Bd. 752, 1971, Seiten 115-135, XP009079695
- WOLFGANG WALTER, REINHARD FRIEDRICH BECKER: LIEBIGS ANNALEN DER CHEMIE, Bd. 755, 1972, Seiten 127-144, XP009079696

## Beschreibung

Die vorliegende Erfindung betrifft neue Carboxamide, mehrere Verfahren zu deren Herstellung und deren Verwendung zur Bekämpfung von schädlichen Mikroorganismen im Pflanzenschutz und Materialschutz.

Es ist bereits bekannt geworden, dass zahlreiche Carboxamide fungizide Eigenschaften besitzen (vgl. z.B. WO 03/070705, EP-A 0 545 099 und JP-A 9-132567). Die Wirksamkeit der dort beschriebenen Stoffe ist gut, lässt aber bei niedrigen Aufwandmengen in manchen Fallen zu wünschen übrig.

Es wurden nun neue Carboxamide der Formel (I) 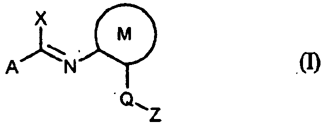 gefunden, in welcher
- X: für OR¹, SR², NHR³, NR⁴R⁵, SOR⁶, SO₂R⁶ steht,
- R¹: C₁-C₁₂-Alkyl, C₂-C₈-Alkenyl, C₂-C₈-Alkinyl, C₁-C₈-Halogenalkyl, Hydroxy-C₁-C₈-alkyl, (C₁-C₄-Alkoxy)-C₁-C₈-alkyl, Bis(C₁-C₄-alkoxy)-C₁-C₈-alkyl, (C₁-C₄-Alkoxy)-carbonyl-(C₁-C₄-alkyl); jeweils gegebenenfalls durch Halogen, C₁-C₄-Alkyl oder C₁-C₄-Halogenalkyl substituiertes Phenyl-(C₁-C₂-alkyl) oder Hetaryl-(C₁-C₂-alkyl); jeweils gegebenenfalls durch C₁-C₄-Alkoxy, Halogen, C₁-C₄-Alkyl oder C₁-C₄-Halogenalkyl substituiertes (C₁-C₆-Alkyl)carbonyl, (C₃-C₈-Cycloalkyl)carbonyl, Phenylcarbonyl oder Hetarylcarbonyl,
- R²: C₁-C₁₂-Alkyl, C₂-C₈-Alkenyl, C₂-C₈-Alkinyl, C₁-C₈-Halogenalkyl, Hydroxy-C₁-C₈-alkyl, (C₁-C4-Alkoxy)-C₁-C₈-alkyl, Bis(C₁-C₄-alkoxy)-C₁-C₈-alkyl, Cyano-(C₁-C₄-alkyl), -CH₂S-CN, (C₁-C₄-Alkoxy)-carbonyl-(C₁-C₄-alkyl); jeweils gegebenenfalls durch Halogen, C₁-C₄-Alkyl oder C₁-C₄-Halogenalkyl substituiertes Phenyl-(C₁-C₂-alkyl) oder Hetaryl-(C₁-C₂-alkyl); jeweils gegebenenfalls durch C₁-C₄-Alkoxy, Halogen, C₁-C₄-Akyl oder C₁-C₄-Halogenalkyl substituiertes (C₁-C₆-Alkyl)carbonyl, (C₃-C₈-Cycloalkyl)carbonyl, Phenylcarbonyl oder Hetarylcarbonyl,
- R³, R⁴ und R⁵: unabhängig voneinander für Amino, Hydroxy, Cyano, C₁-C₁₂-Alkyl, C₁-C₈-Alkoxy, C₂-C₈-Alkenyl, C₂-C₈-Alkinyl, C₁-C₆-Alkylamino, Di(C₁-C₆-alkyl)amino, Hydroxy-C₁-C₈-alkyl, (C₁-C₄-Alkoxy)-C₁-C₈-alkyl, Bis(C₁-C₄-alkoxy)-C₁-C₈-alkyl, Cyano-(C₁-C₄-alkyl), Phenoxy, Phenylamino, Benzyloxy oder Benzylamino stehen,
- R⁶: C₁-C₁₂-Alkyl oder C₁-C₈-Halogenalkyl steht,
- M: für einen jeweils einfach durch R⁷ substituierten Phenyl-, Thiophen-, Pyridin-, Pyrimidin-, Pyridazin oder Pyrazin-Ring oder für einen durch R⁸ substituierten Thiazol-Ring steht,
- R⁷: für Wasserstoff, Fluor, Chlor, Methyl, iso-Propyl, Methylthio oder Trifluormethyl steht,
- R⁸: für Wasserstoff, Methyl, Methylthio oder Trifluormethyl steht,
- Q: für eine direkte Bindung, C₁-C₄-Alkylen, C₂-C₄-Alkenylen, O, S, SO, SO₂ oder NR⁹ steht,
- R⁹: für Wasserstoff, C₁-C₈-Alkyl, C₁-C₄-Allcoxy-C₁-C₄-alkyl, C₁-C₄-Alkylthio-C₁-C₄-alkyl, C₂-C₈-Alkenyl, C₂-C₈-Alkinyl, C₁-C₆-Halogenalkyl, C₂-C₆-Halogenalkenyl, C₂-C₆-Halogen-alkinyl oder C₃-C₆-Cycloalkyl steht,
- Z: für Z¹, Z², Z³, Z⁴, Z⁵ oder Z⁶ steht,
- Z¹: für gegebenenfalls einfach bis fünffach, gleich oder verschieden substituiertes Phenyl steht,
- Z²: für gegebenenfalls einfach bis dreifach, gleich oder verschieden substituiertes Pyridinyl steht,
- Z³: für jeweils gegebenenfalls einfach oder mehrfach, gleich oder verschieden durch Halogen, Alkyl und/oder -(CR¹⁰R¹¹)ₘSiR¹²R¹³R¹⁴ substituiertes Cycloalkyl oder Bicycloalkyl steht,
- Z⁴: für unsubstituiertes C₂-C₂₀-Alkyl oder für einfach oder mehrfach, gleich oder verschieden durch Halogen, Alkylthio, Alkylsulfinyl, Alkylsulfonyl, Alkoxy, Alkylamino, Dialkylamino, Halogenalkylthio, Halogenalkylsulfinyl, Halogenalkylsulfonyl, Halogenalkoxy, Halogenalkylamino, Halogen-dialkylamino, -SiR¹²R¹³R¹⁴ und/oder C₃-C₆-Cycloalkyl substituiertes C₁-C₂₀-Alkyl steht, wobei der Cycloalkylteil seinerseits gegebenenfalls einfach oder mehrfach, gleich oder verschieden durch Halogen und/oder C₁-C₄-Alkyl substituiert sein kann,
- Z⁵: für jeweils gegebenenfalls einfach oder mehrfach, gleich oder verschieden durch Halogen, Alkylthio, Alkylsulfinyl, Alkylsulfonyl, Alkoxy, Alkylamino, Dialkylamino, Halogenalkylthio, Halogenalkylsulfinyl, Halogenalkylsulfonyl, Halogenalkoxy, Halogenalkylamino, Halogen-dialkylamino, -SiR¹²R¹³R¹⁴ und/oder C₃-C₆-Cycloalkyl substituiertes C₂-C₂₀-Alkenyl oder C₂-C₂₀-Alkinyl steht, wobei der Cycloalkylteil seinerseits gegebenenfalls einfach oder mehrfach, gleich oder verschieden durch Halogen und/oder C₁-C₄-Alkyl substituiert sein kann,
- Z⁶: für einen gegebenenfalls einfach oder mehrfach substituierten, gesättigten oder ungesättigten 3- bis 7-gliedrigen Ring steht, welcher ein Siliziumatom als Ringglied enthält, wobei dann Q für eine direkte Bindung oder C₁-C₄-Alkylen steht,
- R¹⁰: für Wasserstoff oder C₁-C₄-Alkyl steht,
- R¹¹: für Wasserstoff oder C₁-C₄-Alkyl steht,
- m: für 0, 1, 2 oder 3 steht,
- R¹² und R¹³: unabhängig voneinander für Wasserstoff, C₁-C₈-Alkyl, C₁-C₈-Alkoxy, C₁-C₄-Alkoxy-C₁-C₄-alkyl, C₁-C₄-Alkylthio-C₁-C₄-alkyl oder C₁-C₆-Halogenalkyl stehen,
- R¹⁴: für Wasserstoff, C₁-C₈-Alkyl, C₁-C₈-Alkoxy, C₁-C₄-Alkoxy-C₁-C₄-alkyl, C₁-C₄-Alkylthio-C₁-C₄-alk-yl, C₂-C₈-Alkenyl, C₂-C₈-Alkinyl, C₁-C₆-Halogenalkyl, C₂-C₆-Halogenalkenyl, C₂-C₆-Halogenalkinyl, C₃-C₆-Cycloalkyl, oder für jeweils gegebenenfalls substituiertes Phenyl oder Phenylalkyl steht,
- M-Q-Z: gemeinsam für jeweils gegebenenfalls einfach bis dreifach durch Methyl substituiertes 1H-2,3-Dihydro-inden-4-yl, 1,3-Dihydro-2-benzofuran-4-yl oder 1,3-Dihydro-2-benzothien-4-yl stehen,
- A: für A1 steht 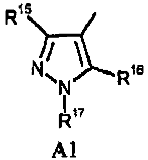
- R¹⁵: für Wasserstoff, Cyano, Halogen, Nitro, C₁-C₄-Alkyl, C₁-C₄-Alkoxy, C₁-C₄-Alkylthio, C₃-C₆-Cycloalkyl. C₁-C₄-Halogenalkyl, C₁-C₄-Halogenalkoxy oder C₁-C₄-Halogenalkylthio mit jeweils 1 bis 5 Halogenatomen, Aminocarbonyl oder Aminocarbonyl-C₁-C₄-alkyl steht,
- R¹⁶: für Wasserstoff, Halogen, Cyano, C₁-C₄-Alkyl, C₁-C₄-Alkoxy oder C₁-C₄-Alkylthio steht,
- R¹⁷: für Wasserstoff, C₁-C₄-Alkyl, Hydroxy-C₁-C₄-alkyl, C₂-C₆-Alkenyl, C₃-C₆-Cycloalkyl, C₁-C₄-Alkylthio-C₁-C₄-alkyl, C₁-C₄-Alkoxy-C₁-C₄-alkyl, C₁-C₄-Halogenalkyl, C₁-C₄-Halogenalkylthio-C₁-C₄-alkyl, C₁-C₄-Halogenalkoxy-C₁-C₄-alkyl mit jeweils 1 bis 5 Halogenatomen, oder Phenyl steht,

Weiterhin wurde gefunden, dass man Carboxamide der Formel (I) nach einem der im Folgenden beschriebenen Verfahren erhält.
(a) Für den Fall, dass X für OR¹ steht, setzt man Carboxamide der Formel (II-a) 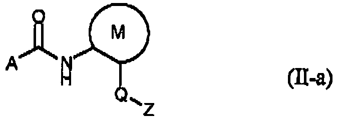 in welcher M, Q, Z und A die oben angegebenen Bedeutungen haben,
   gegebenenfalls in Gegenwart einer Base mit einem Alkylierungsmittel der Formel (III-a)

   LG¹—R¹ (III-a)

   in welcher
   - R¹: die oben angegebenen Bedeutungen hat und
   - LG¹: für eine Abgangsgruppe steht,
   oder mit einem Meerweinsalz der Formel (III-d)

   (R¹)₃O⁺ E⁻ (III-d)

   in welcher
   - R¹: die oben angegebenen Bedeutungen hat und
   - E: für BF₄, SbF₆ oder SbCl₆ steht,
   um und erhält Carboxamide der Formel (I-a)
   um und erhält Carboxamide der Formel (I-a) 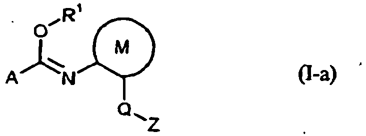 in welcher R¹, M, Q, Z und A die oben angegebenen Bedeutungen haben.
(b) Für den Fall, dass X für SR², SOR⁶ oder SO₂R⁶ steht, setzt man in einem ersten Schritt Carboxamide der Formel (II-a) 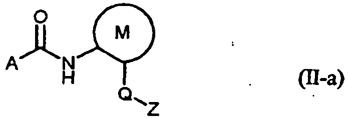 in welcher M, Q, Z und A die oben angegebenen Bedeutungen haben, gegebenenfalls in Gegenwart eines Verdünnungsmittels mit einem Thionierungsmittel um
   und erhält so Carboxamide der Formel (II-b) 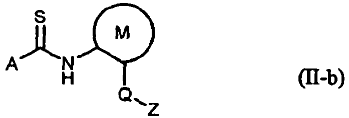 in welcher M, Q, Z und A die oben angegebenen Bedeutungen haben,
   welche man in einem zweiten Schritt gegebenenfalls in Gegenwart einer Base mit einem Alkylierungsmittel der Formel (III-b) oder der Formel (III-c)

   LG²―R² (III-b)

   LG⁶―R⁶ (III-C)

   in welchen
   - R² und R⁶: die oben angegebenen Bedeutungen haben,
   - LG²: für eine Abgangsgruppe steht,
   - LG⁶: für eine Abgangsgruppe steht,
   umsetzt und Carboxamide der Formel (I-b) oder der Formel (I-c) 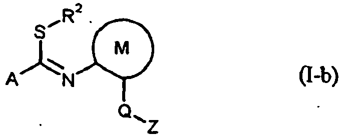 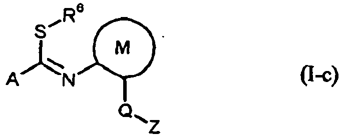 erhält, in welchen R², R⁶, M, Q, Z und A die oben angegebenen Bedeutungen haben,
   und in einem dritten Schritt die Carboxamide der Formel (I-c) anschließend mit einem Oxidationsmittel gegebenenfalls in Gegenwart eines Verdünnungsmittels, gegebenenfalls in Gegenwart eines Säurebindemittels und gegebenenfalls in Gegenwart eines Katalysators umsetzt und so die Carboxamide der Formel (I-d) 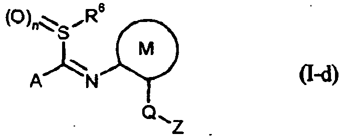 erhält, in welcher
   - R⁶, M, Q, Z und A: die oben angegebenen Bedeutungen haben und
   - n: für 1 oder 2 steht.
(c) Für den Fall, dass X für NHR³ oder NR⁴R⁵ steht, setzt man Carboxamide der Formel (II-b) 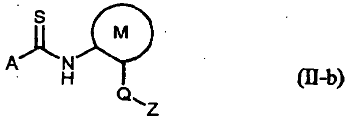 in welcher M, Q, Z und A die oben angegebenen Bedeutungen haben,
   gegebenenfalls in Gegenwart einer Base mit einem Amidierungsmittel der Formel (IV-a) oder der Formel (IV-b) 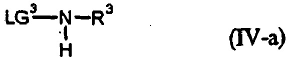 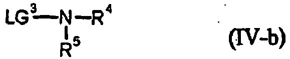 in welchen
   - R³, R⁴ und R⁵: die oben angegebenen Bedeutungen haben und
   - LG³: für eine Abgangsgruppe steht,
   um und erhält Carboxamide der Formel (I-e) oder der Formel (I-f) 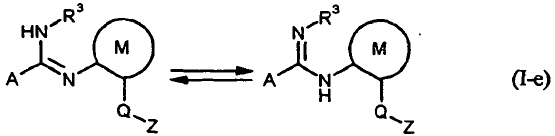 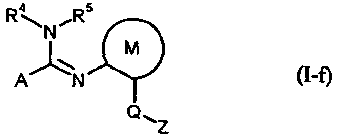 in welchen R³, R⁴, R⁵, M, Q, Z und A die oben angegebenen Bedeutungen haben.

Schließlich wurde gefunden, dass die neuen Carboxamide der Formel (I) sehr gute mikrobizide Eigenschaften besitzen und zur Bekämpfung unerwünschter Mikroorganismen sowohl im Pflanzenschutz als auch im Materialschutz verwendbar sind.

Die erfindungsgemäßen Verbindungen können gegebenenfalls als Mischungen verschiedener möglicher isomerer Formen, insbesondere von Stereoisomeren, wie z. B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die erfindungsgemäßen Biphenylthiazolcarboxamide sind durch die Formel (I) allgemein definiert. Bevorzugte Restedefinitionen der vorstehenden und nachfolgend genannten Formeln sind im Folgenden angegeben. Diese Definitionen gelten für die Endprodukte der Formel (I) wie für alle Zwischenprodukte gleichermaßen.
- X: steht bevorzugt für OR¹.
- X: steht auch bevorzugt für SR².
- X: steht auch bevorzugt für NHR³ oder NR⁴R⁵.
- X: steht auch bevorzugt für SOR⁶ oder SO₂R⁶.

- R¹: steht bevorzugt für C₁-C₈-Alkyl, C₂-C₆-Alkenyl, C₂-C₆-Alkinyl, C₁-C₄-Halogenalkyl, Hydroxy-C₁-C₆-alkyl, (C₁-C₂-Alkoxy)-C₁-C₆-alkyl, Bis(C₁-C₂-alkoxy)-C₁-C₆-alkyl, (C₁-C₂-Alkoxy)-carbonyl-(C₁-C₂-alkyl); jeweils gegebenenfalls durch Fluor, Chlor, Brom, C₁-C₄-Alkyl oder C₁-C₄-Halogenalkyl substituiertes Benzyl, Phenyl-1-ethyl, Phenyl-2-ethyl, Pyridinyl-(C₁-C₂-alkyl) oder Thienyl-(C₁-C₂-alkyl); jeweils gegebenenfalls durch C₁-C₄-Alkoxy, Fluor, Chlor, Brom, C₁-C₄-Alkyl oder C₁-C₄-Halogenalkyl substituiertes (C₁-C₄-Alkyl)-carbonyl, (C₃-C₆-Cycloalkyl)carbonyl, Phenylcarbonyl, Pyridinylcarbonyl oder Thienylcarbonyl.
- R¹: steht besonders bevorzugt für Methyl, Ethyl, n-, iso-Propyl, n-, i-, s-, t-Butyl, Allyl, Propargyl, Trifluormethyl, Difluormethyl, Trichlormethyl, Dichlormethyl, -CH₂CF₃, Pentafluorethyl, Hydroxymethyl, Hydroxyethyl, Hydroxypropyl, Methoxymethyl, Methoxyethyl, Ethoxymethyl, Ethoxyethyl, Dimethoxymethyl, Dimethoxyethyl, Diethoxymethyl, Diethoxyethyl, Methoxycarbonylmethyl, Methotrycarbonylethyl, Ethoxycarbonylmethyl, Ethoxycarbonylethyl; jeweils gegebenenfalls durch Fluor, Chlor, Brom, Methyl oder Trifluormethyl substituiertes Benzyl, Phenyl-1-ethyl, Phenyl-2-ethyl, Pyridinyl-(C₁-C₂-alkyl) oder Zhienyl-(C₁-C₂-alkyl); jeweils gegebenenfalls durch Methoxy, Ethoxy, Fluor, Chlor, Brom, Methyl, Trifluormethyl substituiertes Methylcarbonyl, Ethylcarbonyl, Cyclopropylcarbonyl, Cyclohexylcarbonyl, Phenylcarbonyl, Pyridinylcarbonyl oder Thienylcarbonyl.
- R¹: steht ganz besonders bevorzugt für Methyl, Ethyl, n-Propyl, n-Butyl, n-Hexyl, n-octyl, n-Decyl, n-Dodecyl, Allyl, Propargyl, Trifluormethyl, Trichlormethyl, -CH₂CF₃, Pentafluorethyl, Hydroxymethyl, Hydroxyethyl, Hydroxypropyl, Methoxymethyl, Methoxyethyl, Ethoxymethyl, Ethoxyethyl, Dimethoxymethyl, Dimethoxyethyl, Diethoxymethyl, Diethoxyethyl, Methoxycarbonylmethyl, Methoxycarbonylethyl, Ethoxycarbonylmethyl, Ethoxycarbonylethyl; jeweils gegebenenfalls durch Fluor, Chlor, Brom, Methyl oder Trifluormethyl substituiertes Benzyl, Phenyl-1-ethyl, Phenyl-2-ethyl, Pyridinyl-(C₁-C₂-alkyl) oder Thienyl-(C₁-C₂-alkyl) (hervorgehoben 6-Chlor-3-pyridinyl-methyl, 2,6-Dichlor-4-pyridinylmethyl, 4,5-Dibrom-2-thienyl); jeweils gegebenenfalls durch Methoxy, Ethoxy, Fluor, Chlor, Brom, Methyl, Trifluormethyl substituiertes Methylcarbonyl, Ethylcarbonyl, Cyclopropylcarbonyl, Cyclohexylcarbonyl, Phenylcarbonyl, Pyridinylcarbonyl oder Thienylcarbonyl (hervorgehoben 6-Chlor-3-pyridinylcarbonyl, 2,6-Dichlor-4-pyridinylcarbonyl, 4,5-Dibrom-2-thienylcarbonyl).
- R²: steht bevorzugt für C₁-C₈-Alkyl, Dodecyl, C₂-C₆-Alkenyl, C₂-C₆-Alkinyl, C₁-C₄-Halogenalkyl, Hydroxy-C₁-C₆-alkyl, (C₁-C₂-Alkoxy)-C₁-C₆-alkyl, Bis(C₁-C₂-alkoxy)-C₁-C₆-alkyl, Cyano-(C₁-C₄-alkyl), -CH₂S-CN, (C₁-C₂-Alkoxy)-carbonyl-(C₁-C₂-alkyl); jeweils gegebenenfalls durch Fluor, Chlor, Brom, C₁-C₄-Alkyl oder C₁-C₄-Halogenalkyl substituiertes Benzyl, Phenyl-1-ethyl, Phenylethyl, Pyridinyl-(C₁-C₂-alkyl) oder Thienyl-(C₁-C₂-alkyl); jeweils gegebenenfalls durch C₁-C₄-Alkoxy, Fluor, Chlor, Brom, C₁-C₄-Alkyl oder C₁-C₄-Halogenalkyl substituiertes (C₁-C₄-Alkyl)carbonyl, (C₃-C₆-Cycloalkyl)carbonyl, Phenylcarbonyl, Pyridinylcarbonyl oder Thienylcarbonyl.
- R²: steht besonders bevorzugt für Methyl, Ethyl, n-, iso-Propyl, n-, i-, s-, t-Butyl, n-Hexyl, n-Octyl, n-Decyl, Dodecyl, Allyl, Propargyl, Trifluormethyl, Difluormethyl, Trichlormethyl, Dichlormethyl, -CH₂CF₃, Pentafluorethyl, Hydroxymethyl, Hydroxyethyl, Hydroxypropyl, Methoxymethyl, Methoxyethyl, Ethoxymethyl, Ethoxyethyl, Dimethoxymethyl, Dimethoxyethyl, Diethoxymethyl, Diethoxyethyl, Cyanomethyl, Cyanoethyl, -CH₂S-CN, Methoxycarbonylmethyl, Methoxycarbonylethyl, Ethoxycarbonylmethyl, Ethoxycarbonylethyl; jeweils gegebenenfalls durch Fluor, Chlor, Brom, Methyl oder Trifluormethyl substituiertes Benzyl, Phenyl-1-ethyl, Phenyl-2-ethyl, Pyridinyl-(C₁-C₂-alkyl) oder Thienyl-(C₁-C₂-alkyl); jeweils gegebenenfalls durch Methoxy, Ethoxy, Fluor, Chlor, Brom, Methyl, Trifluormethyl substituiertes Methylcarbonyl, Ethylcarbonyl, Cyclopropylcarbonyl, Cyclohexylcarbonyl, Phenylcarbonyl, Pyridinylcarbonyl oder Thienylcarbonyl.
- R²: steht ganz besonders bevorzugt für Methyl, Ethyl, n-Propyl, n-Butyl, n-Hexyl, n-Octyl, n-Decyl, n-Dodecyl, Allyl, Propargyl, Trifluormethyl, Trichlormethyl, -CH₂CF₃, Pentafluorethyl, Hydroxymethyl, Hydroxyethyl, Hydroxypropyl, Methoxymethyl, Methoxyethyl, Ethoxymethyl, Ethoxyethyl, Dimethoxymethyl, Dimethoxyethyl, Diethoxymethyl, Diethoxyethyl, Cyanomethyl, Cyanoethyl, -CH₂S-CN, Methoxycarbonylmethyl, Methoxycarbonylethyl, Ethoxycarbonylmethyl, Ethoxycarbonylethyl; jeweils gegebenenfalls durch Fluor, Chlor, Brom, Methyl oder Trifluormethyl substituiertes Benzyl, Phenyl-1-ethyl, Phenyl-2-ethyl, Pyridinyl-(C₁-C₂-alkyl) oder Thienyl-(C₁-C₂-alkyl) (hervorgehoben 6-Chlor-3-pyridinyl-methyl, 2,6-Dichlor-4-pyridinylmethyl, 4,5-Dibrom-2-thienyl); jeweils gegebenenfalls durch Methoxy, Ethoxy, Fluor, Chlor, Brom, Methyl, Trifluormethyl substituiertes Methylcarbonyl, Ethylcarbonyl, Cyclopropylcarbonyl, Cyclohexylcarbonyl, Phenylcarbonyl, Pyridinylcarbonyl oder Thienylcarbonyl (hervorgehoben 6-Chlor-3-pyridinylcarbonyl, 2,6-Dichlor-4-pyridinylcarbonyl, 4,5-Dibrom-2-thienylcarbonyl).
- R³, R⁴ und R⁵: stehen unabhängig voneinander bevorzugt für Wasserstoff, Amino, Hydroxy, Cyano, C₁-C₈-Alkyl, C₁-C₆-Alkoxy, C₂-C₆-Alkenyl, C₂-C₆-Alkinyl, C₁-C₄-Alkylamino, Di(C₁-C₄-alkyl)amino, Hydroxy-C₁-C₆-alkyl, (C₁-C₂-Alkoxy)-C₁-C₆-alkyl, Bis(C₁₋C₂-alkoxy)-C₁-C₆-alkyl, Cyano-(C₁-C₄-alkyl), Phenoxy, Phenylamino, Benzyloxy oder Benzylamino.
- R³, R⁴ und R⁵: stehen unabhängig voneinander besonders bevorzugt für Wasserstoff, Amino, Hydroxy, Cyano, Methyl, Ethyl, n-, iso-Propyl, n-, i-, s-, t-Butyl, Methoxy, Ethoxy, n-, iso-Propoxy, n-, i-, s-, t-Butoxy, Allyl, Propargyl, Methylamino, Ethylamino, n-, iso-Propylamino, n-, i-, s-, t-Butylamino, Dimethylamino, Diethylamino, Hydroxymethyl, Hydroxyethyl, Hydroxypropyl, Methoxymethyl, Methoxyethyl, Ethoxymethyl, Ethoxyethyl, Dimethoxymethyl, Dimethoxyethyl, Diethoxymethyl, Diethoxyethyl, Cyanomethyl, Phenoxy, Phenylamino, Benzyloxy oder Benzylamino.
- R⁶: steht bevorzugt für C₁-C₄-Alkyl oder C₁-C₄-Halogenalkyl.
- R⁶: steht besonders bevorzugt für Methyl, Ethyl, n-, iso-Propyl, n-, i-, s-, t-Butyl, Trifluormethyl, Difluormethyl, Trichlormethyl, Dichlormethyl, -CH₂CF₃, Pentafluorethyl.
- R⁶: steht ganz besonders bevorzugt für Methyl, Ethyl, Trifluormethyl, Trichlormethyl, -CH₂CF₃, Pentafluorethyl.
- M: steht bevorzugt für einen der folgenden Cyclen 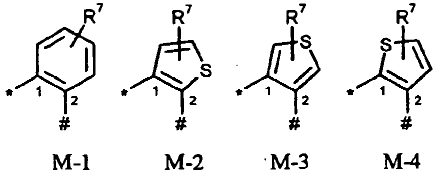 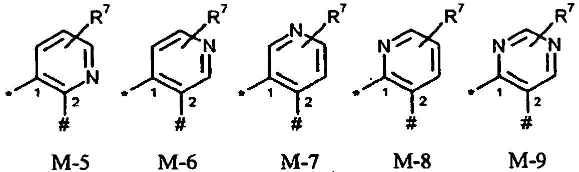 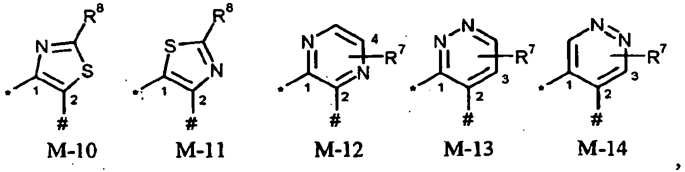 wobei die mit "*" markierte Bindung mit dem Amid, die mit "#" markierte Bindung mit dem Rest R verknüpft ist.
- M: steht besonders bevorzugt für einen Cyclus ausgewählt aus M-1, M-2, M-3, M-4, M-5, M-6, M-9, M-10 und M-11.
- M: steht ganz besonders bevorzugt für einen Cyclus ausgewählt aus M-1, M-2, M-5, M-6, M-9, M-10 und M-11.
- M: steht insbesondere bevorzugt für den Cyclus M-1.
- M: steht außerdem insbesondere bevorzugt für den Heterocyclus M-2.
- M: steht außerdem insbesondere bevorzugt für den Heterocyclus M-5.
- M: steht außerdem insbesondere bevorzugt für den Heterocyclus M-6.
- M: steht außerdem insbesondere bevorzugt für den Heterocyclus M-9.
- M: steht außerdem insbesondere bevorzugt für den Heterocyclus M-10.
- M: steht außerdem insbesondere bevorzugt für den Heterocyclus M-11.
- R⁷: steht bevorzugt für Wasserstoff.
- R⁷: steht für den Fall, dass M für M-1 steht, außerdem bevorzugt für Fluor, wobei Fluor besonders bevorzugt in 4-, 5- oder 6-Position, ganz besonders bevorzugt in 4- oder 6-Position, insbesondere in 4-Position des Anilidrestes steht.
- R⁷: steht für den Fall, dass M für M-1 steht, außerdem bevorzugt für Chlor, wobei Chlor besonders bevorzugt in 5-Position des Anilidrestes steht.
- R⁷: steht für den Fall, dass M für M-1 steht, außerdem bevorzugt für Methyl, wobei Methyl besonders bevorzugt in 3-Position des Anilidrestes steht.
- R⁷: steht für den Fall, dass M für M-1 steht, außerdem bevorzugt für Trifluormethyl, wobei Trifluormethyl besonders bevorzugt in 4- oder 5-Position des Anilidrestes steht.
- R⁷: steht für den Fall, dass M für M-2, M-3 oder M-4 steht, außerdem bevorzugt für Chlor, wobei Chlor besonders bevorzugt in 5-Position (M-2, M-3) oder in 3-Position (M-4) steht.
- R⁷: steht für den Fall, dass M für M-2, M-3 oder M-4 steht, außerdem bevorzugt Für Methyl, wobei Methyl besonders bevorzugt in 5-Position (M-2, M-3) oder in 3-Position (M-4) steht.
- R⁷: steht für den Fall, dass M für M-5, M-6, M-7 oder M-8 steht, außerdem bevorzugt für Fluor, wobei Fluor besonders bevorzugt in 6-Position (M-5, M-6) oder in 3-Position (M-7, M-8) steht.
- R⁷: steht für den Fall, dass M für M-5, M-6, M-7 oder M-8 steht, außerdem Bevorzugt für Chlor, wobei Chlor besonders bevorzugt in 6-Position (M-5, M-6) oder in 3-Position (M-7, M-8) steht.
- R⁷: steht für den Fall, dass M für M-5, M-6, M-7 oder M-8 steht, außerdem bevorzugt für Methyl, wobei Methyl besonders bevorzugt in 4-Position (M-5) oder in 3-Position (M-6, M-7, M-8) steht.
- R⁷: steht für den Fall, dass M für M-9 steht, außerdem bevorzugt für Methyl, wobei Methyl besonders bevorzugt in 3-Position steht.
- R⁷: steht für den Fall, dass M für M-9 steht, außerdem bevorzugt für Trifluormethyl, wobei Trifluormethyl besonders bevorzugt in 3-Position steht.
- R⁷,: steht für den Fall, dass M für M-12 steht, außerdem bevorzugt für Methyl, wobei Methyl besonders bevorzugt in 4-Position steht.
- R⁷: steht für den Fall, dass M für M-12 steht, außerdem bevorzugt für Trifluormethyl, wobei Trifluormethyl besonders bevorzugt in 4-Position steht.
- R⁷: steht für den Fall, dass M für M-13 steht, außerdem bevorzugt für Methyl, wobei Methyl besonders bevorzugt in 3-Position steht.
- R⁷: steht für den Fall, dass M für M-13 steht, außerdem bevorzugt für Trifluormethyl, wobei Trifluormethyl besonders bevorzugt in 3-Position steht.
- R⁷: steht für den Fall, dass M für M-14 steht, außerdem bevorzugt für Methyl, wobei Methyl besonders bevorzugt in 3-Position steht.
- R⁷: steht für den Fall, dass M für M-14 steht, außerdem bevorzugt für Trifluormethyl, wobei Trifluormethyl besonders bevorzugt in 3-Position steht.
- R⁸: steht bevorzugt für Wasserstoff.
- R⁸: steht außerdem bevorzugt für Methyl.
- R⁸: steht außerdem bevorzugt für Trifluormethyl.
- Q: steht bevorzugt für eine direkte Bindung.
- Q: steht außerdem bevorzugt für -CH₂-, -(CH₂)₂-, -(CH₂)₃-, -CH(CH₃)-, -C(CH₃)₂-, besonders bevorzugt für -CH₂-, -(CH₂)₂-, -CH(CH₃)-.
- Q: steht außerdem bevorzugt für -CH=CH-, -CH₂-CH=CH-, -CH(CH₃)-CH=CH-, besonders bevorzugt für -CH=CH-, -CH₂-CH=CH-.
- Q: steht außerdem bevorzugt für O.
- Q: steht außerdem bevorzugt für S.
- Q: steht außerdem bevorzugt für SO.
- Q: steht außerdem bevorzugt für SO₂.
- Q: steht außerdem bevorzugt für NR⁹, besonders bevorzugt für NH.
- R⁹: steht bevorzugt für Wasserstoff C₁-C₆-Akyl, C₁-C₃-Alkoxy-C₇-C₃-alkyl, C₁-C₃-Akylthio-C₁-C₃-alkyl oder C₃-C₆-Cycloalkyl.
- R⁹: steht besonders bevorzugt für Wasserstoff, Methyl, Ethyl, n- oder iso-Propyl, n-, sec-, iso - oder tert-Butyl, Methoxymethyl, Ethoxymethyl, Methoxyethyl, Ethoxyethyl, Methylthiomethyl, Ethylthiomethyl, Methylthioethyl, Ethylthioethyl oder Cyclopropyl.
- R⁹: steht ganz besonders bevorzugt für Wasserstoff, Methyl, Ethyl, n- oder iso-Propyl, iso- oder tert-Butyl, Methoxymethyl oder Methylthiomethyl.
- Z: steht bevorzugt für Z¹.
- Z¹: steht bevorzugt für gegebenenfalls einfach bis fünffach, gleich oder verschieden substituiertes Phenyl, wobei die Substituenten jeweils aus der Liste W¹ ausgewählt sind.
- Z¹: steht besonders bevorzugt für einfach substituiertes Phenyl, wobei die Substituenten aus der Liste W¹ ausgewählt sind.
- Z¹: steht auch besonders bevorzugt für zweifach, gleich oder verschieden substituiertes Phenyl, wobei die Substituenten aus der Liste W¹ ausgewählt sind.
- Z¹: steht auch besonders bevorzugt für dreifach, gleich oder verschieden substituiertes Phenyl, wobei die Substituenten aus der Liste W¹ ausgewählt sind.
- Z¹: steht ganz besonders bevorzugt für einfach in 4-Position substituiertes Phenyl, wobei die Substituenten aus der Liste W¹ ausgewählt sind.
- Z¹: steht ganz besonders bevorzugt für zweifach, gleich oder verschieden in 3,4-Position substituiertes Phenyl, wobei die Substituenten aus der Liste W¹ ausgewählt sind.
- Z¹: steht ganz besonders bevorzugt für zweifach, gleich oder verschieden in 2,3-Position substituiertes Phenyl, wobei die Substituenten aus der Liste W¹ ausgewählt sind.
- Z¹: steht ganz besonders bevorzugt für zweifach, gleich oder verschieden in 2,4-Position substituiertes Phenyl, wobei die Substituenten aus der Liste W¹ ausgewählt sind.
- Z¹: steht ganz besonders bevorzugt für zweifach, gleich oder verschieden in 3,5-Position substituiertes Phenyl, wobei die Substituenten aus der Liste W¹ ausgewählt sind.
- Z¹: steht ganz besonders bevorzugt für dreifach, gleich oder verschieden in 2,4,6-Position substituiertes Phenyl, wobei die Substituenten aus der Liste W¹ ausgewählt sind.
- W¹: steht für Halogen, Cyano, Nitro, Amino, Hydroxy, Formyl, Carboxy, Carbamoyl, Thiocarbamoyl;
jeweils geradkettiges oder verzweigtes Alkyl, Hydroxyalkyl, Oxoalkyl, Alkoxy, Alkoxyalkyl, Alkylthioalkyl, Dialkoxyalkyl, Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylaminocarbonyl, Dialkylaminocarbonyl, Arylalkylaminocarbonyl, Dialkylaminocarbonyloxy mit 1 bis 6 Kohlenstoffatomen in den jeweiligen Kohlenwasserstoffketten, Alkenylcarbonyl oder Alkinylcarbonyl mit 2 bis 6 Kohlenstoffatomen in den jeweiligen Kohlenwasserstoffketten;
Cycloalkyl oder Cycloalkyloxy mit jeweils 3 bis 6 Kohlenstoffatomen;
jeweils gegebenenfalls einfach bis vierfach, gleich oder verschieden durch Fluor, Chlor, Oxo, Methyl, Trifluormethyl oder Ethyl substituiertes, jeweils zweifach verknüpftes Alkylen mit 3 oder 4 Kohlenstoffatomen, Oxyalkylen mit 2 oder 3 Kohlenstoffatomen oder Dioxyalkylen mit 1 oder 2 Kohlenstoffatomen;
oder die Gruppierungen -(CR¹⁰R¹¹)ₘSiR¹²R¹³R¹⁴ oder -C(Q²)=N-Q³, worin
- Q²: für Wasserstoff, Hydroxy oder Alkyl mit 1 bis 4 Kohlenstoffatomen, Halogenalkyl mit 1 bis 4 Kohlenstoffatomen und 1 bis 9 Fluor-, Chlor- und/oder Bromatomen oder Cycloalkyl mit 1 bis 6 Kohlenstoffatomen steht und
- Q³: für Hydroxy, Amino, Methylamino, Phenyl, Benzyl oder für jeweils gegebenenfalls durch Cyano, Hydroxy, Alkoxy, Alkylthio, Alkylamino, Dialkylamino oder Phenyl substituiertes Alkyl oder Alkoxy mit 1 bis 4 Kohlenstoffatomen, oder für Alkenyloxy oder Alkinyloxy mit jeweils 2 bis 4 Kohlenstoffatomen steht,
sowie jeweils gegebenenfalls im Ringteil einfach bis dreifach durch Halogen, und/ oder geradkettiges oder verzweigtes Alkyl oder Alkoxy mit 1 bis 4 Kohlenstoffatomen substituiertes Phenyl, Phenoxy, Phenylthio, Benzoyl, Benzoylethenyl, Cinnamoyl, Heterocyclyl oder Phenylalkyl, Phenylalkyloxy, Phenylalkylthio, oder Heterocyclylalkyl, mit jeweils 1 bis 3 Kohlenstoffatomen in den jeweiligen Alkylteilen.
- W¹: steht bevorzugt für Fluor, Chlor, Brom, Methyl, Ethyl, n- oder i-Propyl, n-, i-, s- oder t-Butyl, Methoxy, Ethoxy, n- oder i-Propoxy, Trifluormethyl, Trifluorethyl, Difluormethoxy, Trifluormethoxy, Difluorchlormethoxy, Trifluorethoxy, jeweils zweifach verknüpftes Difluormethylendioxy oder Tetrafluorethylendioxy,
oder die Gruppierungen -CH₂Si(CH₃)₃, -Si(CH₃)₃ oder -C(Q²)=N-Q³, worin
- Q²: für Wasserstoff, Methyl, Ethyl oder Trifluormethyl steht und
- Q³: für Hydroxy, Methoxy, Ethoxy, Propoxy oder Isopropoxy steht.
- Z: steht bevorzugt für Z².
- Z²: steht bevorzugt für jeweils gegebenenfalls einfach bis dreifach, gleich oder verschieden substituiertes 2-Pyridinyl, 3-Pyridinyl oder 4-Pyridinyl, wobei die Substituenten jeweils aus der Liste W² ausgewählt sind.
- Z²: steht besonders bevorzugt für jeweils einfach substituiertes 2-Pyridinyl, 3-Pyridinyl oder 4-Pyridinyl, wobei die Substituenten jeweils aus der Liste W² ausgewählt sind.
- Z²: steht auch besonders bevorzugt für jeweils zweifach; gleich oder verschieden substituiertes 2-Pyridinyl, 3-Pyridinyl oder 4-Pyridinyl, wobei die Substituenten jeweils aus der Liste W² ausgewählt sind.
- Z²: steht auch besonders bevorzugt für dreifach, gleich oder verschieden substituiertes 2-Pyridinyl, 3-Pyridinyl oder 4-Pyridinyl, wobei die Substituenten jeweils aus der Liste W² ausgewählt sind.
- Z²: steht ganz besonders bevorzugt für einfach in 5-Position substituiertes 2-Pyridinyl oder in 6-Position substituiertes 3-Pyridinyl, wobei die Substituenten jeweils aus der Liste W² ausgewählt sind.
- Z²: steht ganz besonders bevorzugt für zweifach, gleich oder verschieden in 3,5-Position substituiertes 2-Pyridinyl, wobei die Substituenten aus der Liste W² ausgewählt sind.
- Z²: steht ganz besonders bevorzugt für zweifach, gleich oder verschieden in 4,6-Position substituiertes 3-Pyridinyl, wobei die Substituenten aus der Liste W² ausgewählt sind.
- Z²: steht ganz besonders bevorzugt für zweifach, gleich oder verschieden in 3,5-Position substituiertes 4-Pyridinyl, wobei die Substituenten aus der Liste W² ausgewählt sind.
- W²: für Wasserstoff, Halogen, Cyano, Nitro, C₁-C₆-Alkyl, C₂-C₄-Alkenyl, C₂-C₄-Alldnyl, C₁-C₄-Alkoxy, C₁-C₄-Alkylthio, C₁-C₄-Alkylsulphinyl, C₁-C₄-Alkylsulphonyl, C₃-C₆-Cycloalkyl; für C₁-C₄-Halogenalkyl, C₁-C₄-Halogenalkoxy, C₁-C₄-Halogenalkylthio, C₁-C₄-Halogenalkylsulfinyl, C₁-C₄-Halogenalkylsulfonyl mit jeweils 1 bis 5 Halogenatomen; für -SO₂NR⁵²R⁵³, -C(=X)R⁵⁴, -Si(R⁵⁵)₃, C₂-C₄-Alkenylen-Si(R⁵⁵)₃, C₂-C₄-Alkinylen-Si(R⁵⁵)₃, -NR⁵²R⁵³ ,-CH₂-NR⁵²R⁵³ stehen, worin
- R⁵²: für Wasserstoff, C₁-C₄-Alkyl oder -C(=X)R⁵⁴ steht,
- R⁵³: für Wasserstoff, C₁-C₄-Alkyl oder -C(=X)R⁵⁴ steht,
- R⁵² und R⁵³: außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR⁵⁶ enthalten kann,
- R⁵⁴: für Wasserstoff, C₁-C₄-Alkyl, C₁-C₄-Alkoxy oder -NR⁵⁷R⁵⁸ steht,
- R⁵⁵: für Wasserstoff, C₁-C₈-Alkyl, C₁-C₈-Alkoxy, C₁-C₄-Alkoxy-C₁-C₄-alkyl, C₁-C₄-Alkylthio-C₁-C₄-alkyl oder C₁-C₆-Halogenalkyl steht, wobei die drei Reste R⁵⁵ jeweils gleich oder verschieden sein können,
- R⁵⁶: für Wasserstoff oder C₁-C₆-Alkyl steht,
- R⁵⁷: für Wasserstoff oder C₁-C₄-Alkyl steht,
- R⁵⁸: für Wasserstoff oder C₁-C₄-Akyl steht,
- R⁵⁷ und R⁵⁸: außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR⁵⁶ enthalten kann,
- W²: steht bevorzugt für Wasserstoff, Fluor, Chlor, Brom, Cyano, Nitro, Methyl, Ethyl, n- oder iso-Propyl, n-, iso-, sec- oder tert-Butyl, Allyl, Propargyl, Methoxy, Ethoxy, n- oder iso-Propoxy, n-, iso-, sec- oder tert-Butoxy, Methylthio, Ethylthio, n- oder iso-Propylthio, n-, iso-, sec- oder tert-Butylthio, Methylsulphinyl, Ethylsulphinyl, n- oder iso-Propylsulphinyl, n-, iso-, sec- oder tert-Butylsulphinyl, Methylsulphonyl, Ethylsulphonyl, n- oder iso-Propylsulphonyl, n-, iso-, sec- oder tert-Butylsulphonyl, Cyclopropyl, Cyclopentyl, Cyclohexyl, Trifluormethyl, Difluormethyl, Trichlormethyl, Trifluorethyl, Trifluormethoxy, Difluormethoxy, Trichlormethoxy, Difluormethylthio, Difluorchlormethylthio, Trifluormethylthio, Trifluormethylsulfinyl, Trifluormethylsulfonyl, -SO₂NMe₂, -C(=X)R⁵⁴, -Si(R⁵⁵)₃, - CH=CH-Si(R⁵⁵)₃, -CH₂-CH=CH-Si(R⁵⁵)₃, -CH=CH-CH₂-Si(R⁵⁵)₃, -C≡C-Si(R⁵⁵)₃, -CH₂-C≡C-Si(R⁵⁵)₃, -C≡C-CH₂-Si(R⁵⁵)₃, -CH₂-C≡C-CH₂-Si(R⁵⁵)₃, -NR⁵²R⁵³, -CH₂-NR⁵²R⁵³_{.}
- R⁵²: steht bevorzugt für Wasserstoff, Methyl, Ethyl, n- oder iso-Propyl oder -C(=X)R⁵⁴.
- R⁵²: steht besonders bevorzugt für Wasserstoff oder Methyl.
- R⁵³: steht bevorzugt für Wasserstoff, Methyl, Ethyl, n- oder iso-Propyl oder -C(=X)R⁵⁴.
- R⁵³: steht besonders bevorzugt für Wasserstoff oder Methyl.
- R⁵² und R⁵³: bilden außerdem gemeinsam mit dem Stickstoffatom, an das sie gebunden sind, bevorzugt einen gegebenenfalls einfach bis vierfach, gleich oder verschieden durch Fluor, Chlor, Brom oder Methyl substituierten gesättigten Heterocyclus aus der Reihe Morpholin, Thiomorpholin oder Piperazin, wobei das Piperazin am zweiten Stickstoffatom durch R⁵⁶ substituiert sein kann.
- R⁵⁴: steht bevorzugt für Wasserstoff, Methyl, Ethyl, n- oder iso-Propyl, Methoxy, Ethoxy, n-oder iso-Propoxy oder -NR⁵⁷R⁵⁸.
- R⁵⁴: steht besonders bevorzugt für Wasserstoff, Methyl, Ethyl, Methoxy, Ethoxy oder -NR⁵⁷R⁵⁸.
- R⁵⁵: steht bevorzugt für Methyl, Ethyl, Methoxy, Ethoxy, Methoxymethyl, Ethoxymethyl, Methoxymethyl, Ethoxyethyl, Methylthiomethyl, Ethylthiomethyl, Methylthioethyl oder Ethylthioethyl, wobei die drei Reste R⁵⁵ jeweils gleich oder verschieden sein können.
- R⁵⁵: steht besonders bevorzugt für Methyl, Methoxy, Methoxymethyl oder Methylthiomethyl, wobei die drei Reste R⁵⁵ jeweils gleich oder verschieden sein können.
- R⁵⁵: steht ganz besonders bevorzugt für Methyl.
- R⁵⁶: steht bevorzugt für Wasserstoff oder C₁-C₄-Alkyl.
- R⁵⁶: steht besonders bevorzugt für Wasserstoff, Methyl, Ethyl, n- oder iso-Propyl, n-, iso-, sec - oder tert-Butyl.
- R⁵⁷: steht bevorzugt für Wasserstoff, Methyl, Ethyl, n- oder iso-Propyl.
- R⁵⁷: steht besonders bevorzugt für Wasserstoff oder Methyl.
- R⁵⁸: steht bevorzugt für Wasserstoff, Methyl, Ethyl, n- oder iso-Propyl.
- R⁵⁸: steht besonders bevorzugt für Wasserstoff oder Methyl.
- R⁵⁷ und R⁵⁸: bilden außerdem gemeinsam mit dem Stickstoffatom, an das sie gebunden sind, bevorzugt einen gegebenenfalls einfach bis vierfach, gleich oder verschieden durch Fluor, Chlor, Brom oder Methyl substituierten gesättigten Heterocyclus aus der Reihe Morpholin, Thiomorpholin oder Piperazin, wobei das Piperazin am zweiten Stickstoffatom durch R⁷ substituiert sein kann.
- Z: steht auch bevorzugt für Z³.
- Z³: steht bevorzugt für jeweils gegebenenfalls einfach bis vierfach, gleich oder verschieden durch Halogen, C₁-C₄-Alkyl, -CH₂Si(CH₃)₃ und/oder -Si(CH₃)₃ substituiertes Cycloalkyl oder Bicycloalkyl mit jeweils 3 bis 10 Kohlenstoffatomen.
- Z³: steht besonders bevorzugt für jeweils gegebenenfalls einfach bis vierfach, gleich oder verschieden durch Chlor, Methyl, -CH₂Si(CH₃)₃ und/oder -Si(CH₃)₃ substituiertes Cyclopropyl, Cyclopentyl, Cyclohexyl, Cycloheptyl, Cyclooctyl, Cyclononyl, Bicyclo[2.2.1]heptyl oder Bicyclo[2.2.2]octyl.
- Z³: steht ganz besonders bevorzugt für durch Chlor und Methyl substituiertes Cyclopropyl.
- Z: steht auch bevorzugt für Z⁴.
- Z⁴: steht bevorzugt für unsubstituiertes C₂-C₂₀-Alkyl oder für einfach oder mehrfach, gleich oder verschieden durch Fluor, Chlor, Brom, Iod, C₁-C₆-Alkylthio, C₁-C₆ Alkylsulfinyl, C₁-C₆-Alkylsulfonyl, C₁-C₆-Alkoxy, C₁-C₆-Akylamino, Di(C₁-C₆-alkyl)amino, C₁-C₆-Halogenalkylthio, C₁-C₆-Halogenalkylsulfinyl, C₁-C₆-Halogenalkylsulfonyl, C₁-C₆-Halogenalkoxy, C₁-C₆-Halogenallcylamino, Halogen-di(C₁-C₆-alkyl)amino, -SiR¹²R¹³R¹⁴ und/oder C₃-C₆-Cycloalkyl substituiertes C₁-C₂₀-Alkyl, wobei der Cycloalkylteil seinerseits gegebenenfalls einfach bis vierfach, gleich oder verschieden durch Fluor, Chlor, Brom, Iod, C₁-C₄-Alkyl und/oder C₁-C₄-Halogenalkyl substituiert sein kann.
- Z⁴: steht besonders bevorzugt für unsubstituiertes C₂-C₂₀-Alkyl.
- Z⁴: steht auch besonders bevorzugt für durch Fluor, Chlor, Brom, Iod, C₁-C₆-Alkylthio. C₁-C₄-Alkylsulfinyl, C₁-C₄-Alkylsulfonyl, C₁-C₄-Alkoxy, C₁-C₄-Alkylamino, Di(C₁-C₄-alkyl)amino, C₁-C₄-Halogenalkylthio, C₁-C₄-Halogenalkylsulfinyl, C₁-C₄-Halogenalkylsulfonyl, C₁-C₄-Halogenalkoxy, C₁-C₄-Halogenalkylamino, Halogen-di(C₁-C₄-alkyl)amino mit jeweils 1 bis 9 Fluor-, Chlor - und/oder Bromatomen, -SiR⁶R⁷R⁸, Cyclopropyl, Dichlorcyclopropyl, Cyclobutyl, Cyclopentyl oder Cyclohexyl substituiertes C₁-C₂₀-Alkyl; ganz besonders bevorzugt für durch Fluor, Chlor, Methylthio, Ethylthio, n- oder iso-Propylthio, n-, iso-, sec-, tert-Butylthio, Pentylthio, Hexylthio, Methylsulfonyl, Ethylsulfonyl, n- oder iso-Propylsulfonyl, n-, iso-, sec-, tert-Butylsulfonyl, Methoxy, Ethoxy, n- oder iso-Propoxy, n-, iso-, sec-, tert-Butoxy, Methylamino, Ethylamino, n-oder iso-Propylamino, n-, iso-, sec-, tert-Butylamino, Dimethylamino, Diisopropylamino, Trifluormethylthio, Trifluormethoxy, -SiR¹²R¹³R¹⁴, Cyclopropyl, Dichlorcyclopropyl, Cyclobutyl, Cyclopentyl oder Cyclohexyl substituiertes C₁-C₂₀-Alkyl.
- Z: steht auch bevorzugt für Z⁵.
- Z⁵: steht bevorzugt für jeweils gegebenenfalls einfach oder mehrfach, gleich oder verschieden durch Fluor, Chlor, Brom, Iod, C₁-C₆-Alkylthio, C₁-C₆-Alkylsulfinyl, C₁-C₆-Alkylsulfonyl, C₁-C₆-Alkoxy, C₁-C₆-Alkylamino, Di(C₁-C₆-alkyl)amino, C₁-C₆-Halogenalkylthio, C₁-C₆ Halogenalkylsulfinyl, C₁-C₆-Halogenalkylsulfonyl C₁-C₆-Halogenalkoxy, C₁-C₆-Halogenalkylamino, Halogen-di(C₁-C₆-alkyl)amino, -SiR¹²R¹³R¹⁴ und/oder C₃-C₆-Cycloalkyl substituiertes C₂-C₂₀-Alkenyl oder C₂-C₂₀-Alkinyl, wobei der Cycloalkylteil seinerseits gegebenenfalls einfach bis vierfach, gleich oder verschieden durch Fluor, Chlor, Brom, Iod, C₁-C₄-Alkyl und/oder C₁-C₄-Halogenalkyl substituiert sein kann.
- Z⁵: steht besonders bevorzugt für jeweils gegebenenfalls durch Fluor, Chlor, Brom, Iod. C₁-C₆-Alkylthio, C₁-C₄-Alkylsulfinyl, C₁-C₄-Alkylsulfonyl, C₁-C₄-Alkoxy, C₁-C₄-Alkylamino, Di(C₁-C₄-alkyl)amino, C₁-C₄-Halogenalkylthio, C₁-C₄-Halogenalkylsulfinyl, C₁-C₄-Halogenalkylsulfonyl, C₁-C₄-Halogenalkoxy, C₁-C₄-Halogenalkylamino, Halogen-di(C₁-C₄-alkyl)amino mit jeweils 1 bis 9 Fluor-, Chlor- und/oder Bromatomen, -SiR¹²R¹³R¹⁴, Cyclopropyl, Dichlorcyclopropyl, Cyclobutyl, Cyclopentyl oder Cyclobexyl substituiertes C₁-C₂₀-Alkenyl oder C₂-C₂₀-Alkinyl.
- Z⁵: steht ganz besonders bevorzugt für C₂-C₂₀-Alkenyl oder C₂-C₂₀-Alkinyl.
- Z: steht auch bevorzugt für Z⁶.
- Z⁶: steht bevorzugt für einen der folgenden Ringe
- R¹⁰: steht besonders bevorzugt für Wasserstoff.
- R¹¹: steht bevorzugt für Wasserstoff oder Methyl.
- R¹¹: steht besonders bvorzugt für Wasserstoff.
- m: steht bevorzugt für 0, 1 oder 2.
- R¹² und R¹³: stehen unabhängig voneinander bevorzugt für C₁-C₆-Alkyl, C₁-C₆-Alkoxy, C₁-C₃-Alkoxy-C₁-C₃-alkyl oder C₁-C₃-Alkylthio-C₁-C₃-alkyl.
- R¹² und R¹³: stehen unabhängig voneinander besonders bevorzugt für Methyl, Ethyl, Methoxy, Ethoxy, Methoxymethyl, Ethoxymethyl, Methoxyethyl, Ethoxyethyl, Methylthiomethyl, Ethylthiomethyl, Methylthioethyl oder Ethylthioethyl.
- R¹² und R¹³: stehen unabhängig voneinander ganz besonders bevorzugt für Methyl, Methoxy, Methoxymethyl oder Methylthiomethyl.
- R¹² und R¹³: stehen insbesondere bevorzugt jeweils für Methyl,
- R¹⁴: steht bevorzugt für C₁-C₆-Alkyl, C₁-C₆-Alkoxy, C₁-C₃-Alkoxy-C₁-C₃-alkyl, C₁-C₃-Alkylthio-C₁-C₃-alkyl, C₃-C₆-Cycloalkyl, Phenyl oder Benzyl.
- R¹⁴: steht besonders bevorzugt für Methyl, Ethyl, n- oder iso-Propyl, n-, sec-, iso- oder tert-Butyl, Methoxy, Ethoxy, n- oder iso-Propoxy, n-, sec-, iso- oder tert-Butoxy, Methoxymethyl, Ethoxymethyl, Methoxyethyl, Ethoxyethyl, Methylthiomethyl, Ethylthiomethyl, Methylthioethyl, Ethylthioethyl, Cyclopropyl, Phenyl oder Benzyl,
- R¹⁴: sheht ganz besonders bevorzugt für Methyl, Ethyl, n- oder iso-Propyl, iso- oder tert-Butyl, Methoxy, iso-Propoxy, iso- oder tert-Butoxy, Methoxymethyl, Methylthiomethyl oder Phenyl,
- R¹⁴: steht insbesondere bevorzugt für Methyl, Ethyl, n- oder iso-Propyl, iso- oder tert-Butyl, Methoxy, iso-Propoxy, iso- oder tert-Butoxy.
- R¹⁴: steht hervorgehoben für Methyl,
- M-Q-Z: stehen auch bevorzugt gemeinsam für 1,1,3-Trimethyl-1H-2,3-dihydro-inden-4-yl, 1,3-Dimethyl-1H-2,3-dihydro-inden-4-yl, 1,1,3-Trimethyl-1,3-dihydro-2-benzofuran-4-yl, 1,3-Dimethyl-1,3-dihydro-2-benzofuran-4-yl, 1,1,3-Trimethyl-1,3-dihydro-2-benzothien-4-yl oder 1,3-Dimethyl-1,3-dihydro-2-benzothien-4-yl.
- M-Q-Z: stehen auch besonders bevorzugt gemeinsam für 1,1,3-Trimethyl-1H-2,3-dihydro-inden-4-yl.
- R¹⁵: steht bevorzugt für Wasserstoff, Cyano, Fluor, Chlor, Brom, Iod, Methyl, Ethyl, iso-Propyl, Methoxy, Ethoxy, Methylthio, Ethylthio, Cyclopropyl, C₁-C₂-Halogenalkyl, C₁-C₂-Halogenalkoxy mit jeweils 1 bis 5 Fluor, Chlor und/oder Bromatomen, Trifluormethylthio, Difluormethylthio, Aminocarbonyl, Aminocarbonylmethyl oder Aminocarbonylethyl.
- R¹⁵: steht besondors bevorzugt für Wasserstoff, Fluor, Chlor, Brom, Iod, Methyl, Ethyl, iso-Propyl, Monofluormethyl, Monofluorethyl, Difluormethyl, Trifluormethyl, Difluorchlormethyl, Trichlormethyl, Dichlormethyl, Cyclopropyl, Methoxy, Ethoxy, Trifluonnethoxy, Trichlormethoxy, Methylthio, Ethylthio, Trifluormethylthio oder Difluormethylthio.
- R¹⁵: steht ganz besonders bevorzugt für Wasserstoff, Fluor, Chlor, Brom, Iod, Methyl, iso-Propyl, Monofluormethyl, Monofluorethyl, Difluormethyl, Trifluormethyl, Difluorchlormethyl oder Trichlormethyl.
- R¹⁵: steht insbesondere bevorzugt für Methyl, Difluormethyl, Trifluormethyl oder 1 -Fluorethyl.
- R¹⁶: steht bevorzugt für Wasserstoff, Fluor, Chlor, Brom, Iod, Methyl, Ethyl, Methoxy, Ethoxy, Methylthio oder Ethylthio.
- R¹⁶: steht besonders bevorzugt für Wasserstoff, Fluor, Chlor, Brom, Iod oder Methyl.
- R¹⁶: steht ganz besonders bevorzugt für Wasserstoff, Fluor, Chlor oder Methyl.
- R¹⁷: steht bevorzugt für Wasserstoff, Methyl, Ethyl, n-Propyl, iso-Propyl, C₁-C₂-Halogenalkyl mit 1 bis 5 Fluor, Chlor und/oder Bromatomen, Hydroxymethyl, Hydroxyethyl, Cyclopropyl, Cyclopentyl, Cyclohexyl oder Phenyl.
- R¹⁷: steht besonders bevorzugt für Wasserstoff, Methyl, Ethyl, iso-Propyl, Trifluormethyl, Difluormethyl, Hydroxymethyl, Hydroxyethyl oder Phenyl,
- R¹⁷: steht ganz besonders bevorzugt für Wasserstoff, Methyl, Trifluormethyl oder Phenyl,
- R¹⁷: steht insbesondere bevorzugt für Methyl.

Bevorzugt sind solche Verbindungen der Formel (I), in welcher alle Reste jeweils die oben genannten bevorzugten Bedeutungen haben.
Besonders bevorzugt sind solche Verbindungen der Formel (I), in welcher alle Reste jeweils die oben genannten besonders bevorzugten Bedeutungen haben.

Bevorzugt und jeweils als Teilmenge der oben genannten Verbindungen der Formel (I) zu verstehen sind folgende Gruppen von neuen Carboxamiden:

### Gruppe 1: Carboxamide der Formel (I-a)

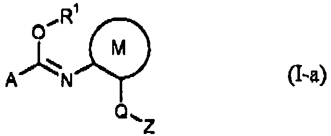

in welcher R¹, M, Q, Z und A die oben angegebenen Bedeutungen haben.

### Gruppe 2: Carboxamide der Formel (I-b)

### Gruppe 1: Carboxamide der Formel (I-a)

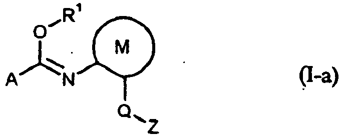

in welcher R¹, M, Q, Z und A die oben angegebenen Bedeutungen haben.

### Gruppe 2: Carboxamide der Formel (I-b)

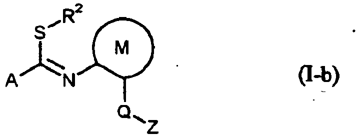

in welcher R², M, Q, Z und A die oben angegebenen Bedeutungen haben.

### Gruppe 3: Carboxamide der Formel (I-e)

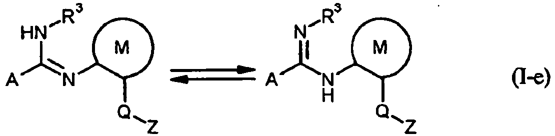

in welcher R³, M, Q, Z und A die oben angegebenen Bedeutungen haben.

### Gruppe 4: Carboxamide der Formel (I-f)

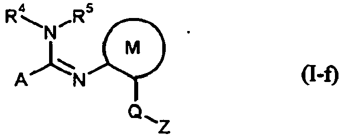

in welcher R⁴, R⁵, M, Q, Z und A die oben angegebenen Bedeutungen haben.

### Gruppe 5: Carboxamide der Formel (I-d)

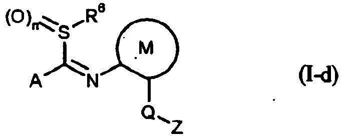

in welcher n, R⁶, M, Q, Z und A die oben angegebenen Bedeutungen haben n steht bevorzugt für 2.

Bevorzugt sind Carboxamide der Formel (I) sowie der Gruppen 1 bis 5, in welcher M für M-1 steht.
Bevorzugt sind Carboxamide der Formel (I) sowie der Gruppen 1 bis 5, in welcher M für M-2 steht.
Bevorzugt sind Carboxamide der Formel (I) sowie der Gruppen 1 bis 5, in welcher M für M-5 steht.
Bevorzugt sind Carboxamide der Formel (I) sowie der Gruppen 1 bis 5, in welcher M für M-6 steht.
Bevorzugt sind Carboxamide der Formel (I) sowie der Gruppen 1 bis 5, in welcher M für M-9 steht.
Bevorzugt sind Carboxamide der Formel (I) sowie der Gruppen 1 bis 5, in welcher M für M-10 steht.
Bevorzugt sind Carboxamide der Formel (I) sowie der Gruppen 1 bis 5, in welcher M für M-11 steht.
Bevorzugt sind Carboxamide der Formel (I) sowie der Gruppen 1 bis 5, in welcher Q für eine direkte Bindung und Z für Z¹ steht.
Bevorzugt sind Carboxamide der Formel (I) sowie der Gruppen 1 bis 5, in welcher Q für eine direkte Bindung und Z für Z⁴ steht.

Z⁴ steht insbesondere bevorzugt für die Gruppierung 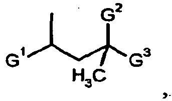 worin
- G¹: für Wasserstoff oder Methyl steht,
- G²: für Wasserstoff oder Methyl steht,
- G³: für Methyl oder Ethyl steht.
Z⁴ steht auch bevorzugt für eine der Gruppierungen G1 bis G8 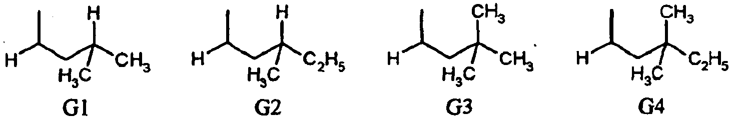 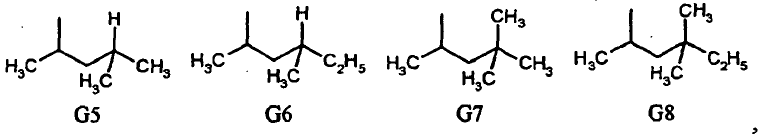 besonders bevorzugt für G3, G5 oder G7.

Die Definition C₁-C₂₀-Alkyl umfasst den größten hierin definierten Bereich für einen Alkylrest. Im Einzelnen umfasst diese Definition die Bedeutungen Methyl, Ethyl, n-, iso-Propyl, n-, iso-, sec-, tert-Butyl, sowie jeweils alle isomeren Pentyle, Hexyle, Heptyle, Octyle, Nonyle, Decyle, Undecyle, Dodecyle, Tridecyle, Tetradecyle, Pentadecyle, Hexadeeyle, Heptadecyle, Octadecyle, Nonadeeyle und Eicosyle. Ein bevorzugter Bereich ist C₂-C₁₂-Alkyl wie Ethyl und geradkettiges oder verzweigtes Propyl, Butyl, Pentyl, Hexyl, Heptyl, Octyl, Nonyl, Decyl, Undecyl und Dodecyl, besonders geradkettiges oder verzweigtes C₃-C₁₀-Alkyl wie Propyl, 1-Methylethyl, Butyl, 1-Methylpropyl, 2-Methylpropyl, 1,1-Dimethylethyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-3-methylpropyl, n-Heptyl, 1-Methylhexyl, 1-Ethylpentyl, 2-Ethylpentyl, 1-Propylbutyl, Octyl, 1-Methylheptyl, 2-Methylheptyl, 1-Ethylhexyl, 2-Ethylhexyl, 1-Propylpentyl, 2-Propylpentyl, Nonyl, 1-Methyloctyl, 2-Methyloctyl, 1-Ethylheptyl, 2-Ethylheptyl, 1-Propylhexyl, 2-Propylhexyl, Decyl, 1-Methylnonyl, 2-Methylnonyl, 1-Ethyloctyl, 2-Ethyloctyl, 1-Propylheptyl und 2-Propylheptyl, insbesondere Propyl, 1-Methylethyl, Butyl, 1-Methylbutyl, 2-Methylbutyl, 3-Methylbutyl, 1,1-Dimethylethyl, 1,2-Dimethylbutyl, 1,3-Dimethylbutyl, Pentyl, 1-Methylbutyl, 1-Ethylpropyl, Hexyl, 3-Methylpentyl, Heptyl, 1-Methylhexyl, 1-Ethyl-3-methylbutyl, 1-Methylheptyl, 1,2-Dimethylhexyl, 1,3-Dimethyloctyl, 4-Methyloctyl, 1,2,2,3-Tetramethylbutyl, 1,3,3-Trimethylbutyl, 1,2,3-Trimethylbutyl, 1,3-Dimethylpentyl, 1,3-Dimethylhexyl, 5-Methyl-3-hexyl, 2-Methyl-4-heptyl, 2,6-Dimethyl-4-heptyl und 1-Methyl-2-cyclopropylethyl

Durch Halogen substituiertes Alkyl steht beispielsweise für Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 3-Chlor-1-methylbutyl, 2-Chlor-1-methylbutyl, 1-Chlorbutyl, 3,3-Dichlor-1-methylbutyl, 3-Chlor-1-methylbutyl, 1-Methyl-3-trifluormethylbutyl, 3-Methyl-1-trifluormethylbutyl.

Der Substituent -SiR¹²R¹³R¹⁴ steht bevorzugt für folgende Reste: SiMe₃, SiMe₂Et, SiMe₂CHMe₂, SiMe₂CH₂CHMe₂, SiMe₂CH₂CMe₃, SiMe₂OCHMe₂, SiMe₂OCH₂CHMe₂, SiMe₂OMe, SiMe₂CMe₃, SiMe₂CH₂CH₂Me.

Die Definition C₂-C₂₀-Alkenyl umfasst den größten hierin definierten Bereich für einen Alkenylrest. Im Einzelnen umfasst diese Definition die Bedeutungen Ethenyl, n-, iso-Propenyl, n-, iso-, sec-, tert-Butenyl, sowie jeweils alle isomeren Pentenyle, Hexenyle, Heptenyle, Octenyle, Nonenyle, Decenyle, Undecenyle, Dodecenyle, Tridecenyle, Tetradecenyle, Pentadecenyle, Hexadecenyle, Heptadecenyle, Octadecenyle, Nonadecenyle und Eicosenyle, 1-Methyl-1-propenyl, 1-Ethyl-1-butenyl, 2,4-Dimethyl-1-pentenyl, 2,4-Dimethyl-2-pentenyl.

Die Definition C₂-C₂₀-Alkinyl umfasst den größten hierin definierten Bereich für einen Alkinylrest. Im Einzelnen umfasst diese Definition die Bedeutungen Ethinyl, n-, iso-Propinyl, n-, iso-, sec-, tert-Butinyl, sowie jeweils alle isomeren Pentinyle, Hexinyle, Heptinyle, Octinyle, Noninyle, Decinyle, Undecinyle, Dodecinyle, Tridecinyle, Tetradecinyle, Pentadecinyle, Hexadecinyle, Heptadecinyle, Octadecinyle, Nonadecinyle und Eicosinyle.

Gegebenenfalls substituierte Reste können einfach oder mehrfach substituiert sein, wobei bei Mehrfachsubstitutionen die Substituenten gleich oder verschieden sein können. So schließt die Definition Dialkylamino auch eine unsymmebssch durch Alkyl substituierte Aminogruppe wie z.B. Methyl-ethylamino ein.

Durch Halogen substituierte Reste, wie z.B. Halogenalkyl, sind einfach oder mehrfach Halogeniert. Bei mehrfacher Halogenierung können die Halogenatome gleich oder verschieden sein. Halogen steht dabei für Fluor, Chlor, Brom und Iod, insbesondere für Fluor, Chlor und Brom.

Die erfindungsgemäßen Verfahren lassen sich durch das folgende allgemeine Reaktionsschema veranschaulichen:

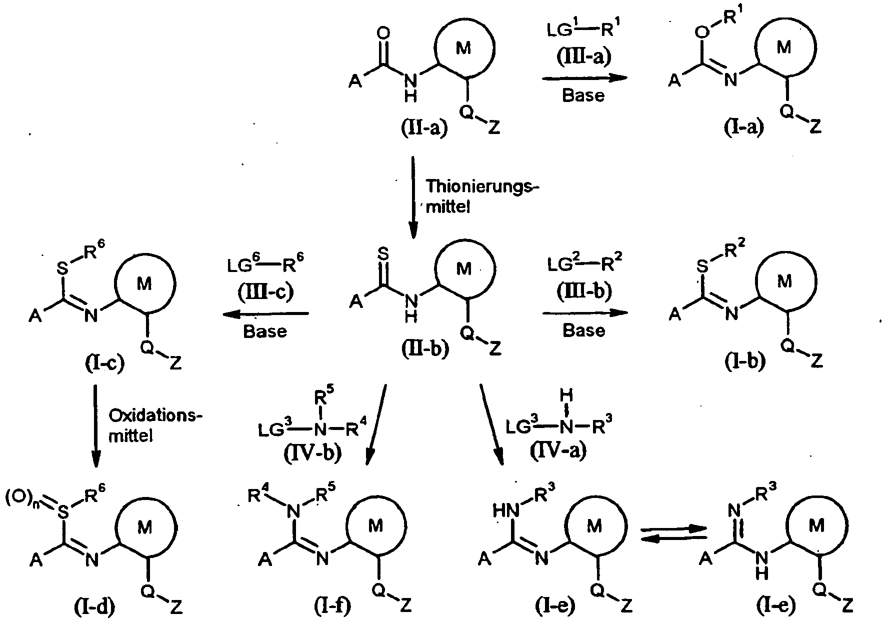

Die zur Durchfühnung des erfindungsgemäßen Verfahrens (a) sowie des ersten Schrittes des erfindungsgmäßen Verfahrens (b) als Ausgangsstoffe benötigten Carboxamide sind durch die Formel (II-a) allgemein definiert. In dieser Formel **(II-a)** stehen M, Q, Z und A bevorzugt, besonders (III-a) steht R¹ bevorzugt, besonders bevorzugt, ganz besonders bevorzugt bzw. insbesondere bevorzugt für diejenigen Bedeutungen, die bereits in Zusammenhang mit der Beschreibung der erfindungsgemäßen Stoffe der Formel (I) als bevorzugt, besonders bevorzugt usw. für diesen Rest angegeben wurden. LG¹ steht für eine Abgangsgruppe, bevorzugt für Halogen, Triflat (Trifluormethylsulfonat), Tosylat (p-Toluolsulfonat), besonders bevorzugt für Chlor, Brom, Iod, Triflat (Trifluormethylsulfonat), Tosylat (p-Toluolsulfonat).

Alkylierungsmittel der Formel (III-a) sind bekannt.

Die weiterhin zur Durchführung des erfindungsgemäßen Verfahrens (a) als Ausgangsstoffe benötigten Meerweinsalze sind durch die Formel (III-d) allgemein definiert. In dieser Formel (III-d) steht R¹ bevorzugt, besonders bevorzugt, ganz besonders bevorzugt bzw. insbesondere bevorzugt für diejenigen Bedeutungen, die bereits in Zusammenhang mit der Beschreibung der erfindungsgemäßen Stoffe der Formel (I) als bevorzugt, besonders bevorzugt usw. für diesen Rest angegeben wurden.

Meerweinsalze der Formel (III-d) sind bekannt.

Die bei der Durchführung des erfindungsgemäßen Verfahrens (b) als Zwischenprodukte auftretenden bzw. bei der Durchführung des erfindungsgemäßen Verfahrens (c) als Ausgangsstoffe benötigten Carboxamide sind durch die Formel (II-b) allgemein definiert. In dieser Formel (II-b) stehen M, Q, Z und A bevorzugt, besonders bevorzugt, ganz besonders bevorzugt bzw. insbesondere bevorzugt für diejenigen Bedeutungen, die bereits in Zusammenhang mit der Beschreibung der erfindungsgemäßen Stoffe der Formel (I) als bevorzugt, besonders bevorzugt usw. für diese Reste angegeben wurden.

Die Carboxamide der Formel (II-b) sind teilweise bekannt und/oder lassen sich nach bekannten Verfahren herstellen (vgl. z.B. WO 01/42223).

Die weiterhin zur Durchführung des erfindungsgemäßen Verfahrens (b) als Ausgangsstoffe benötigten Alkylierungsmittel sind durch die Formel (III-b) und die Formel (III-c) allgemein definiert. In diesen Formeln (III-b) und (III-c) stehen R² und R⁶ bevorzugt, besonders bevorzugt, ganz besonders bevorzugt bzw. insbesondere bevorzugt für diejenigen Bedeutungen, die bereits in Zusammenhang mit der Beschreibung der erfindungsgemäßen Stoffe der Formel (I) als bevorzugt, besonders bevorzugt usw. für diese Reste angegeben wurden. LG² steht für eine Abgangsgruppe, bevorzugt für Halogen, Triflat (Trifluormethylsulfonat), Tosylat (p-Toluolsulfonat), besonders bevorzugt für Chlor, Brom, Iod, Triflat (Trifluormethylsulfonat), Tosylat (p-Toluolsulfonat). LG⁶ steht für eine Abgangsgruppe, bevorzugt für Halogen, Triflat (Trifluormethylsulfonat), Tosylat (p-Toluolsulfonat), besonders bevorzugt für Chlor, Brom, Iod, Triflat (Trifluormethylsulfonat), Tosylat (p-Toluolsulfonat).

Alkylierungsmittel der Formel (III-b) und der Formel (III-c) sind bekannt.

Halogen, Triflat (Trifluormethylsulfonat), Tosylat (p-Toluolsulfonat), besonders bevorzugt für Chlor, Brom, Iod, Triflat (Trifluormethylsulfonat), Tosylat (p-Toluolsulfonat). LG⁶ steht für eine Abgangsgruppe, bevorzugt für Halogen, Triflat (Trifluormethylsulfonat), Tosylat (p-Toluolsulfonat), besonders bevorzugt für Chlor, Brom, Iod, Triflat (Trifluormethylsulfonat), Tosylat (p-Toluolsulfonat).

Alkylierungsmittel der Formel (III-b) und der Formel (III-c) sind bekannt.

Die weiterhin zur Durchführung des erfindungsgemäßen Verfahrens (c) als Ausgangsstoffe benötigten Amidierungsmittel sind durch die Formel (IV-a) und die Formel (IV-b) allgemein definiert. In diesen Formeln (IV-a) und (IV-b) stehen R³, R⁴ und R⁵ bevorzugt, besonders bevorzugt, ganz besonders bevorzugt bzw. insbesondere bevorzugt für diejenigen Bedeutungen, die bereits in Zusammenhang mit der Beschreibung der erfindungsgemäßen Stoffe der Formel (I) als bevorzugt, besonders bevorzugt usw. für diese Reste angegeben wurden. LG³ steht für eine Abgangsgruppe, bevorzugt für Wasserstoff, Amino, Hydroxy, Trialkylsilyl, besonders bevorzugt für Wasserstoff, Trimethylsilyl.

Amidierungsmittel der Formel (IV-a) und der Formel (IV-b) sind bekannt.

Als Verdünnungsmittel zur Durchführung der erfindungsgemäßen Verfahren (a) und (c) sowie des zweiten Schrittes des erfindungsgemäßen Verfahrens (b) kommen alle inerten organischen Lösungsmittel in Betracht. Hierzu gehören vorzugsweise aliphatische, alicyclische oder aromatische Kohlenwasserstoffe, wie z.B. Petrolether, Hexan, Heptan, Cyclohexan, Methylcyclohexan, Benzol, Toluol, Xylol oder Decalin; Halogenierte Kohlenwasserstoffe, wie z.B. Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; deren Gemische mit Wasser oder reines Wasser.

Die erfindungsgemäßen Verfahren (a) und (c) sowie der zweite Schritt des erfindungsgemäßen Verfahrens (b) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, hydroxide, -amide, -alkoholate, -acetate, -carbonate oder -hydrogencarbonate, wie z.B. Natriumhydrid, Natriumamid, Lithiumdiisopropylamid, Natrium-methylat, Natrium-ethylat, Kalium-tert.-butylat, Natriumhydroxid, Kaliumhydroxid, Natriumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylinorpholin, N,N-Dimethylaminopyridin, Diazabicyclooctan (DABCO), Diazabicyclononen (DBN) oder Diazabicycloundecen (DBU).

Der erste Schritt des erfindungsgemäßen Verfahrens (b) wird in Gegenwart eines Thionierungsmittels durchgeführt. Als solche kommen alle für solche Reaktionen üblichen Reagenzien mirage. Vorzugsweise verwendbar sind Schwefel, Phosphorpentasulfid (P₂S₅, P₄S₁₀) oder Lawessons Reagenz.

Als Verdünnungsmittel zur Durchführung des erfindungsgemäßen Verfahrens (b) kommen alle inerten organischen Lösungsmittel in Betracht. Hierzu gehören vorzugsweise aliphatische, alicyclische oder aromatische Kohlenwasserstoffe, wie z.B. Petrolether, Hexan, Heptan, Cyclohexan, Methylcyclohexan, Benzol, Toluol, Xylol oder Decalin; Halogenierte Kohlenwasserstoffe, wie z.B. Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; deren Gemische mit Wasser oder reines Wasser.

Als Oxidationsmittel zur Durchführung des dritten Schrittes des erfindungsgemäßen Verfahrens (b) kommen alle üblichen zur Schwefeloxidation verwendbaren Oxidationsmittel infrage. Insbesondere geeignet sind Wasserstoffperoxid, organische Persäuren, wie beispielsweise Peressigsäure, m-Chlorperbenzoesäure, p-Nitroperbenzoesäure oder Luftsauerstoff.

Als Verdünnungsmittel zur Durchführung des dritten Schrittes des erfindungsgemäßen Verfahrens (b) kommen inerte organische Lösungsmittel infrage. Vorzugsweise verwendet man Kohlenwasserstoffe, wie Benzin, Benzol, Toluol, Hexan oder Petrolether; chlorierte Kohlenwasserstoffe, wie Dichlotmethan, 1,2-Dichlorethan, Chloroform, Tetrachlorkohlenstoff oder Chlorbenzol; Ether, wie Diethylether, Dioxan oder Tetrahydrofuran; Carbonsäuren, wie Essigsäure oder Propionsäute; oder dipolare aprotische Lösungsmittel, wie Acetonitril, Aceton, Essigsäureethylester oder Dimethylformamid.

Der dritte Schritt des erfindungsgemäßen Verfahrens (b) kann gegebenenfalls in Gegenwart eines Säurebindemittels durchgeführt werden. Als solche kommen alle üblicherweise verwendbaren organischen und anorganischen Säurebindemittel infrage. Vorzugsweise verwendet man Erdalkali - oder Alkalimetallhydroxide, -acetate oder -carbonate, wie beispielsweise Calciumhydroxid, Natriumhydroxid, Natriumacetat oder Natriumcarbonat.

Der dritte Schritt des erfindungsgemäßen Verfahrens (b) kann gegebenenfalls in Gegenwart eines geeigneten Katalysators durchgeführt werden. Als solche kommen alle üblicherweise für derartige Schwefeloxidationen gebräuchlichen Metallsalz-Katalysatoren infrage. Beispielhaft genannt sei in diesem Zusammenhang Ammoniummolybdat und Natriumwolframat.

Die Reaktionstemperaturen können bei der Durchführung der erfindungsgemäßen Verfahren (a) und (c) sowie des zweiten Schrittes des erfindungsgemäßen Verfahrens (b) in einem größeren Bereich variiert werden. Im Allgemeinen arbeitet man bei Temperaturen von 0°C bis 150°C, vorzugsweise bei Temperaturen von 20°C bis 110°C.

Die Reaktionstemperaturen können bei der Durchführung des dritten Schrittes des erfindungsgemäßen Verfahrens (b) in einem größeren Bereich variiert werden. Im Allgemeinen arbeitet man bei Temperaturen zwischen -20°C und +70°C, vorzugsweise bei Temperaturen zwischen 0°C und +50°C.

Zur Durchführung des erfindungsgemäßen Verfahrens (a) zur Herstellung der Verbindungen der Formel (I-a) setzt man pro Mol an Carboxamid der Formel (II-a) im Allgemeinen 0.8 bis 5 Mol, vorzugsweise 1 bis 3 Mol an Alkylierungsmittel der Formel (III-a) bzw. 0.1 bis 1.5 Mol, bevorzugt 0.25 bis 1 Mol an Meerweinsalz der Formel (III-d) ein.

Zur Durchführung des ersten Schrittes des erfindungsgemäßen Verfahrens (b) zur Herstellung der Verbindungen der Formel (II-b) setzt man auf 1 Mol an Carboxamid der Formel **(II-a)** im Allgemeinen 0,8 bis 1,2 Mol, vorzugsweise äquimolare Mengen Thionierungsmittel ein.

Zur Durchführung des zweiten Schrittes des erfindungsgeanäßen Verfahrens (b) zur Herstellung der Verbindungen der Formel (I-b) bzw. der Formel (I-c) setzt man pro Mol an Carboxamid der Formel (II-b) im Allgemeinen 0,8 bis 5 Mol, vorzugsweise 1 bis 3 Mol an Alkylierungsmittel der Formel **(III-b)** bzw. der Formel **(III-c)** ein.

Zur Durchführung des dritten Schrittes des erfindungsgemäßen Verfahrens (b) zur Herstellung der Verbindungen der Formel (I-d) setzt man auf 1 Mol an Carboxamid der Formel (I-c) im Allgemeinen 0,8 bis 1,2 Mol, vorzugsweise äquimolare Mengen Oxidationsmittel ein, wenn man die Oxidation des Schwefels auf der Sulfoxidstufe unterbrechen will. Zur Oxidation zum Sulfon setzt man pro Mol an Carboxamid der Formel (I-c) im Allgemeinen 1,8 bis 3,0 Mol, vorzugsweise doppelt molare Mengen an Oxidationsmittel ein.

Zur Durchführung des erfindungsgemäßen Verfahrens (c) zur Herstellung der Verbindungen der Formel (I-e) bzw. der Formel (I-f) setzt man pro Mol an Carboxamid der Formel (II-b) im Allgemeinen 0,8 bis 5 Mol, vorzugsweise 1 bis 3 Mol an Amidierungsmittel der Formel (IV-a) bzw. der Formel (IV-b) ein.

Die erfindungsgemäßen Verfahren werden im Allgemeinen unter Normaldruck durchgeführt. Es ist jedoch auch möglich, unter erhöhtem oder vermindertem Druck - im Allgemeinen zwischen 0,1 bar und 10 bar - zu arbeiten.

Die erfindungsgemäßen Stoffe weisen eine starke mikrobizide Wirkung auf und können zur Bekämpfung von unerwünschten Mikroorganismen, wie Pilze und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Erkrankungen, hervorgerufen durch Erreger des Echten Mehltaus wie z.B.
Blumeria-Arten, wie beispielsweise Blumeria gaminis;
Podosphaera-Arten, wie beispielsweise Podosphaera leucotricha;
Sphaerotheca-Arten, wie beispielsweise Sphaerotheca fuliginea;
Uncinula-Arten, wie beispielsweise Uncinula necator;

Erkrankungen, hervorgerufen durch Erreger von Rostkrankheiten wie z.B.
Gymnosporangium-Arten, wie beispielsweise Gymnosporangium sabinae
Hemileia-Arten, wie beispielsweise Hemileia vastatrix;
Phakopsora-Arten, wie beispielsweise Phakopsora pachyrhizi und Phakopsora meibomiae;
Puccinia-Arten, wie beispielsweise Puccinia recondita oder Puccinia gaminis;
Uromyces-Arten, wie beispielsweise Uromyces appendiculatus;

Erkrankungen, hervorgerufen durch Erreger der Gruppe der Oomyceten wie z.B.
Bremia-Arten, wie beispielsweise Bremia lactucae;
Peronospora-Arten, wie beispielsweise Peronospora pisi oder P. brassicae;
Phytophthora-Arten, wie beispielsweise Phytophthora infestans;
Plasmopara-Arten, wie beispielsweise Plasmopara viticola;
Pseudoperonospora-Arten, wie beispielsweise Pseudoperonospora humuli oder Pseudoperonospora cubensis;
Pythium-Arten, wie beispielsweise Pythium ultimum;

Blattfleckenkrankheiten und Blattwelken, hervorgerufen durch z.B.
Alternaria-Arten, wie beispielsweise Alternaria solani;
Cercospora-Arten, wie beispielsweise Cercospora beticola;
Cladosporium-Arten, wie beispielsweise Cladosporium cucumerinum;
Cochliobolus-Arten, wie beispielsweise Cochliobolus sativus
(Konidienform: Drechslera, Syn: Helminthosporium);
Colletotrichum-Arten, wie beispielsweise Colletotrichum lindemuthanium;
Cycloconium-Arten, wie beispielsweise Cycloconium oleaginum;
Diaporthe-Arten, wie beispielsweise Diaporthe citri;
Elsinoe-Arten, wie beispielsweise Elsinoe fawcettii;
Gloeosporium-Arten, wie beispielsweise Gloeosporium laeticolor;
Glomerella-Arten, wie beispielsweise Glomerella cingulata;
Guignardia-Arten, wie beispielsweise Guignardia bidwelli;
Leptosphaeria-Arten, wie beispielsweise Leptosphaeria maculans;
Magnaporthe-Arten, wie beispielsweise Magnaporthe grisea;
Mycosphaerella-Arten, wie beispielsweise Mycosphaerella graminicola und Mycosphaerella fijiensis;
Phaeosphaeria-Arten, wie beispielsweise Phaeosphaeria nodorum;
Pyrenophora-Arten, wie beispielsweise Pyrenophora teres;
Ramularia-Arten, wie beispielsweise Ramularia collo-cygni;
Rhynchosporium-Arten, wie beispielsweise Rhynchosporium secalis;
Septoria-Arten, wie beispielsweise Septoria apii;
Typhula-Arten, wie beispielsweise Typhula incarnata;
Venturia-Arten, wie beispielsweise Venturia inaequalis;

Wurzel- und Stengelkrankheiten, hervorgerufen durch z.B.
Corticium-Arten, wie beispielsweise Corticium graminearum;
Fusarium-Arten, wie beispielsweise Fusarium oxysporum;
Gaeumannomyces-Arten, wie beispielsweise Gaeumannomyces graminis;
Rhizoctonia-Arten, wie beispielsweise Rhizoctonia solani;
Tapesia-Arten, wie beispielsweise Tapesia acuformis oder Tapesia yallundae;
Thielaviopsis-Arten, wie beispielsweise Thielaviopsis basicola;

Ähren- und Rispeneriuankungen (inklusive Maiskolben), hervorgerufen durch z.B. Alternaria-Arten, wie beispielsweise Alternaria spp.;

Aspergillus-Arten, wie beispielsweise Aspergillus flavus;
Cladosporium-Arten, wie beispielsweise Cladosporium cladosporioides;
Claviceps-Arten, wie beispielsweise Claviceps purpurea;
Fusarium-Arten, wie beispielsweise Fusarium culmorum;
Gibberella-Arten, wie beispielsweise Gibberella zeae;
Monographella-Arten, wie beispielsweise Monographella nivalis;

Erkrankungen, hervorgerufen durch Brandpilze wie z.B.
Sphacelotheca-Arten, wie beispielsweise Sphacelotheca reiliana;
Tilletia-Arten, wie beispielsweise Tilletia caries;
Urocystis-Arten, wie beispielsweise Urocystis occulta;
Ustilago-Arten, wie beispielsweise Ustilago nuda;

Fruchtfäule hervorgerufen durch z.B.
Aspergillus-Arten, wie beispielsweise Aspergillus flavus;
Botrytis-Arten, wie beispielsweise Botrytis cinerea;
Penicillium-Arten, wie beispielsweise Penicillium expansum und Penicillium purpurogenum;
Sclerotinia-Arten, wie beispielsweise Sclerotinia sclerotiorum;
Verticilium-Arten, wie beispielsweise Verticilium alboatrum;

Samen- und bodenbürtige Fäulen und Welken, sowie Sämlingserkrankungen, hervorgerufen durch z.B.
Fusarium-Arten, wie beispielsweise Fusarium culmorum;
Phytophthora Arten, wie beispielsweise Phytophthora cactorum;
Pythium-Arten, wie beispielsweise Pythium ultimum;
Rhizoctonia-Arten, wie beispielsweise Rhizoctonia solani;
Sclerotium-Arten, wie beispielsweise Sclerotium rolfsii;

Krebserkrankungen, Gallen und Hexenbesen, hervorgerufen durch z.B.
Nectria-Arten, wie beispielsweise Nectiria galligena;

Welkeerkrankungen hervorgerufen durch z.B.
Monilinia-Arten, wie beispielsweise Monilinia laxa;

Deformationen von Blättern, Blüten und Früchten, hervorgerufen durch z.B.
Taphrina-Arten, wie beispielsweise Taphrina deformans;

Degenerationserkrankungen holziger pflanzen, hervorgerufen durch z.B.
Esca-Arten, wie beispielsweise Phaeomoniella chlamydospora und Phaeoacremonium aleophilum und Fomitiporia mediterranea;
Blüten- und Samenerkrankungen, hervorgerufen durch z.B.
Botrytis-Arten, wie beispielsweise Botrytis cinerea;

Erkrankungen von Pflanzenknollen, hervorgerufen durch z.B.
Rhizoctonia-Arten, wie beispielsweise Rhizoctonia solani;
Helminthosporium-Arten, wie beispielsweise Helminthosporium solani;

Erkrankungen, hervorgerufen durch bakterielle Erreger wie z.B.
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;

Bevorzugt können die folgenden Krankheiten von Soja-Bohnen bekämpft werden:
Pilzkrankheiten an Blättern, Stängeln, Schoten und Samen verursacht durch z.B.
Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dacluliophora glycines), Downy Mildew (Peronospora manshurica), Drechslera blight (Drechslera glycini), Frogeye Leaf spot (Cercospora sojina), Leptosphaemlina Leaf Spot (Leptosphaerulina trifolii), Phyllostica Leaf Spot (Phyllostida sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi), Scab (Sphaceloma glycines), Stemphylium Leaf Blight (Stemphylium botryosum), Target Spot (Corynespora cassücola)

Pilzkrankheiten an Wurzeln und der Stängelbasis verursacht durch z.B.
Black Root Rot (Calonecoria crotalariae), Charcoal Rot (Macrophomina phaseolina), Fusarium Blight or Wilt, Root Rot, and Pod and Collar Rot (Fusarimn oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryamm, Pythium myriotylum, Pythium ultimum), Rhizoctonia Root Rot, Stem Decay, and Damping-Off (Rhizoctonia solani), Sclerotinia Stem Decay (Sclerotinia sclerotiomum), Sclerotinia Southem Blight (Sclerotinia rolfsii), Thielaviopsis Root Rot (Thielaviopsis basicola).

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen, bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen von 1 bis 28 Tage, vorzugsweise 1 bis 14 Tage, besonders bevorzugt 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz - und Saatgut, und des Bodens.

Dabei lassen sich die erfindungsgemäßen Wirkstoffe mit besonders gutem Erfolg zur Bekämpfung von Getreidekrankheiten, wie z.B. gegen Puccinia-Arten und von Krankheiten im Wein-, Obst- und Gemüseanbau, wie z.B. gegen Botrytis-, Venturia- oder Altemaria-Arten, einsetzen.

Die erfindungsgemäßen Wirkstoffe eignen sich auch zur Steigerung des Emteertrages. Sie sind außerdem mindertoxisch und weisen eine gute Pflanzenverträglichkeit auf.
Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Im Matenalschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichodenna viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulienmgen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorbeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen infrage:
Fungizide:
   1) Inhibitoren der Nukleinsäuresymhese: z.B. Benalaxyl, Benataxyl-M, Bupirimate, Clozylacon, Dimethirimol, Ethirimol, Furalaxyl, Hymexazol, Mefenoxam, Metalaxyl, Metalaxyl-M, Ofurace, Oxadixyl, Oxolinic acid;
   2) Inhibitoren von Mitose und Zellteilung: z.B. Benomyl, Carbendazim, Diethofencarb, Ethaboxam, Fuberidazole, Pencyeuron, Thiabendazole, Thiophanate-methyl, Zoxamide;
   3) Inhibitoren der Respiration (Atmungsketten-Inhibitoren):
      3.1) Inhibitoren am Komplex I der Atmungskette: z.B. Diflumetorim;
      3.2) Inhibitoren am Komplex II der Atmungskette: z.B. Boscalid/Nicobifen, Carboxin, Fenfuram, Flutolanil, Furametpyr, Furmecyclox, Mepronil, Oxycarboxin, Penthiopyrad, Thifluzamide;
      3.3) Inhibitoren am Komplex III der Atmungskette: z.B. Amisulbrom, Azoxystrobin, Cyazofamid, Dimoxystrobin, Enestrobin, Famoxadone, Fenamidone, Fluoxastrobin, Kresoxim-methyl, Metominostrobin, Orysastrobin, Picoxystrobin, Pyracloscrobin, Trifloxystrobin;
   4) Entkoppler: z.B. Dinocap, Fluazinam, Meptyldinocap;
   5) Inhibitoren der ATP Produktion: z.B. Fentin acetate, Fentin chloride, Fentin hydroxide, Silthiofam;
   6) Inhibitoren der Aminosäure- und Protein-Biosynthese: z.B. Andoprim, Blasticidin-S, Cyprodinil, Kasugamycin, Kasugamycin hydrochloride hydrate, Mepanipyrim, Pyrimethanil;
   7) Inhibitoren der Signaltransduktion: z.B. Fenpiclonil, Fludioxonil, Quinoxyfen;
   8) Inhibitoren der Lipid- und Membran-Synthese: z.B. Biphenyl, Chlozolinate, Edifenphos, Iodocarb, Iprobenfos, Iprodione, Isoprothiolane, Procymidone, Propamocarb, Propamocarb hydrochloride, Pyrazophos, Tolclofos-methyl, Vinclozolin;
   9) Inhibitoren der Ergosterol-Biosynthese: z.B. Aldimorph, Azaconazole, Bitertanol, Bromuconazole, Cyproconazole, Diclobutrazole, Difenoconazole, Diniconazole, Diniconazole-M, Dodemorph, Dodemorph acetate, Epoxiconazole, Etaconazole, Fenarimol, Fenbuconazole, Fenhexamid, Fenpropidin, Fenpropimorph, Fluquinconazole, Flurprimidol, Fhisilazole, Flutriafol, Furconazole, Furconazole-cis, Hexaconazole, Imazalil, Imazalil sulfate, Imibenconazole, Ipconazole, Metconazole, Myclobutanil, Naftifine, Nuarimol, Oxpoconazole, Paclobutrazol, Pefurazoate, Penconazole, Prochloraz, Propiconazole, Prothioconazole, Pyributicarb, Pyrifenox, Simeconazole, Spiroxamine, Tebuconazol, Terbinafine, Tetraconazole, Triadimefon, Triadimenol, Tridemorph, Triflumizole, Triforine, Triticonazole, Uniconazole, Viniconazole, Voriconazole;
   10) Inhibitoren der Zellwandsynthese: z.B. Benthiavalicarb, Dimethomorph, Flumorph, Iprovalicarb, Polyoxins, Polyoxorim, Validamycin A;
   11) Inhibitoren der Melanin-Biosynthese: z.B. Carpropamid, Diclocymet, Fenoxanil, Phthalide, Pyroquilon, Tricyclazole;
   12) Resistenzinduktoren: z.B. Acibenzolar-S-methyl, Probenazole, Tiadinil;
   13) Verbindungen mit Multisite-Aktivität: z.B. Bordeaux Mixture, Captafol, Captan, Chlorothalonil, Copper naphthenate, Copper oxide, Copper oxychloride, Kupferzubereitungen wie z.B. Kupferhydroxid, Kupfersulfat, Dichlofluanid, Dithianon, Dodine, Dodine free base, Ferbam, Fluorofolpet, Folpet, Guazatine, Guazatine acetate, Iminoctadine, Iminoctadine albesilate, Iminoctadine triacetate, Mancopper, Mancozeb, Maneb, Metiram, Metiram Zinc, Oxine-Copper, Propineb, Sulphur und Schweferlzubereitungen wie z.B. Calcium polysulphide, Thiram, Tolylfluanid, Zineb, Ziram;
   14) eine Verbindung aus der folgenden Liste: (2E)-2-(2-{[6-(3-Chlor-2-methylphenoxy)-5-fluorpyrimidin-4-yl]oxy}phenyl)-2-(methoxyimino)-N-methylacetamid, (2E)-2-{2-[({[(1E)-1-(3-{[(E)-1-Fluor-2-phenylvinyl]oxy}phenyl)ethyliden]amino}oxy)methyl]phenyl}-2-(methoxyimino)-N-methylacetamid, 1-(4-Chlorphenyl)-2-(1H-1,2,4-triazol-1-yl)cycloheptanol, 1-[(4-Methoxyphmoxy)methyl]-2,2-dimethylpropyl-1H-imidazol-1-carboxylat, 2-(4-Chlorphenyl)-N-{2-[3-methoxy-4-(prop-2-yn-1-yloxy)phenyl]ethyl}-2-(prop-2-yn-1-yloxy)amid, 2,3,5,6-Tetrachlor-4-(methylsulfonyl)pyridin, 2-Butoxy-6-iod-3-propyl-4H-chromen-4-on, 2-Chlor-N-(1,1,3-trimthyl-2,3-dihydro-1H-inden-4-yl)nicofinamid, 2-Phenylphenol und Salze davon, 3,4,5-Trichlorpyridin-2,6-dicarbonitril, 3,4-Dichlor-N-(2-cyanophenyl)isothiazol-5-carboxamid, 3-[5-(4-Chlorphenyl)-2,3-dimthylisoxazolidin-3-yl]pyridin, 5-Chlor-6-(2,4,6-tritluorophenyl)-N-[(1R)-1,2,2-trimethylpropyl][1,2,4]triazolo[1,5-a]pyrimidin-7-amin, 5-Chlor-7-(4-methylpiperidin-1-yl)-6-(2,4,6-trifluorphenyl)[1,2,4]triazolo[1,5-a]pyrimidin, 5-Chlor-N-[(1R)-1,2-dimethylpropyl]-6-(2,4,6-trifluorphenyl)[1,2,4]triazolo[1,5-a]pyrimidin-7-amin, 8-Hydroxyquinolinsulfat, Benthiazole, Bethoxazin, Capsimycin, Carvone, Chinomethionat, Cufraneb, Cyflufenamid, Cymoxanil, Dazomet, Debacarb, Dichlorophen, Diclomezine, Dicloran, Difenzoquat, Difenzoquat Methylsulpthate, Diphenylamine, Ferimzone, Flumetover, Fluopicolide, Fluoroimide, Flusulfamide, Fosetyl-Aluminium, Fosetyl-Calcium, Fosetyl-Sodium, Hexachlorobenzene, Irumamycin, Methasulfocarb, Methyl (2-chlor-5-{(1E)-N-(6-methylpyridin-2-yl)methoxy]ethanimidoyl}benzyl)carbamat, Methyl (2E)-2-{2-[({cyclopropyl[(4-methoxyphenyl)imino}methyl}thio)methyl]phenyl}-3-methoxyacrylat, Methyl 1-(2,2-dimethyl-2,3-dihydro-1H-inden-1-yl)-1H-imdazol-5-carboxylat, Methyl 3-(4-chlorphenyl)-3-1[N-(isopropoxy-carbonyl)valyl]amino}propanoat, Methyl isothiocyanate, Metrafenone, Mildiomycin, N-(3',4'-Dichlor-5-fluorbiphenyl-2-yl)-3-(difluormethyl)-1-methyl-1H-pyrazol-4-carboxamid, N-(3-Ethyl-3,5,5-trimethylcyclohexyl)-3-(formylamino)-2-hydroxybenzamid, N-(4-Chlor-2-nitrophenyl)-N-ethyl-4-methyl-benzeansulfonamid, N-[(5-Brom-3-chlorpyridin-2-yl)methyl]-2,4-dichlormicotinamid, N-[1-(5-Brom₋3-chlorpyridin-2-yl)ethyl]-2,4-dichlomicotinamid, N-[1-(5-Brom-3-chlorpyridin-2-yl)ethyl]-2-fluor-4-iodnicotinamid, N-[2-(4-{[3-(4-Chlorphenyl)prop-2-yn-1-yl]oxy}-3-methoxyphenyl)ethyl]-N²-(methylsulfonyl)valmamid, N-{(Z)-[(Cyclopropylmethoxy)imino][6-(difluormethoxy)-2,3-difluorphenyl]methyl} -2-phenylacetamid, N-{2-[3-Chlor-5-(trifluormethyl)pyridin-2-yl]ethyl}-2-(trifluromethyl)benzamid, Natamycin, Nickel Dimethyldithiocarbamate, Nitrothal-isopropyl, O-{1-[(4-Methoxyphenoxy)methyl]-2,2-dimethylpropyl} 1H-Imidazol-1-carbothioat, Octhilinone, Oxamocarb, Oxyfenthiin, Pentachlorophenol und Salze, Phosphorsäure und ihre Salze, Piperalin, Propamocarb Fosetylate, Propanosine-Sodium, Proquinazid, Pyrrolnitrine, Quintozene, Tecloftalam, Tecnazene, Triazoxide, Trichlamide, Zarilamid.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

*1. Acetylcholinesterase (AChE) Inhibitoren*
   1.1 Carbamate (z.B. Alanycarb, Aldicarb, Aldoxycarb, Allyxycarb, Aminocarb, Azamethiphos, Bendiocarb, Benfuracarb, Bufiencarb, Butacarb, Butocarboxim, Butoxycarboxim, Carbaryl, Carbofuran, Carbosulfan, Chloethocarb, Coumaphos, Cyanofenphos, Cyanophos, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Triazamate, Trimethacarb, XMC, Xylylcarb)
   1.2 Organophosphate (z.B. Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S-methylsluphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/-ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prodhoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion)
*2. Natrium-Kanal-Modulatoren* / *Spannungsabhängige Natrium-Kanal-Blocker*
   2.1 Pyrethroide (z.B. Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, DDT, Deltamethrin, Empenthrin (1R-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda-Cyhalothrin, Metofluthrin, Permethrin (cis-, trans-), Phenothrin (1R-trans isomer), Prallethrin, Profluthrin, Provifenbute, Pyresmethrin, Resmethrin, RU 15525, Silafluofen, Tau-Fluvalinate, Tefluthrin, Terallethrin, Tetramethrin (1R-isomer), Tralomethrin, Transfluthrin, ZXI 8901, Pyrethrins (pyrethrum))
   2.2 Oxadiazine (z.B. Indoxacarb)
*3. Acetylcholin-Rezeptor-Agonisten*/*-Antagonisten*
   3.1 Chloronicotinyle/Neonicotinoide (z.B. Acetamiprid, Clothianidin, Dinotefuran, Imidacloprid, Nitenpyram, Nithiazine, Thiacloprid, Thiamethoxam)
   3.2 Nicotine, Bensultap, Cartap
*4. Acetylcholin-Rezeptor-Modulatoren*
   4.1 Spinosyne (z.B. Spinosad)
*5. GABA-gesteuerte Chlorid-Kanal-Antagonisten*
   5.1 Cyclodiene Organochlorine (z.B. Camphechlor, Chlordane, Endosulfan, Gamma-HCH, HCH, Heptachlor, Lindane, Methoxychlor
   5.2 Fiprole (z.B. Acetoprole, Ethiprole, Fipronil, Vaniliprole)
*6. Chlorid-Kanal-Aktivatoren*
   6.1 Mectine (z.B. Abamectin, Avermectin, Emamectin, Emamectin-benzoate, Ivermectin, Milbemectin, Milbemycin)
*7. Juvenilhormon-Mimetika*
   (z.B. Diofenolan, Epofenonane, Fenoxycarb, Hydroprene, Kinoprene, Methoprene, Pyriproxifen, Triprene)
*8. Ecdysonagonisten*/*disruptoren*
   8.1 Diacylhydrazine (z.B. Chromafenozide, Halofenozide, Methoxyfenozide, Tebufenozide)
*9. Inhibitoren der Chitinbiosynthese*
   9.1 Benzoylharnstoffe (z.B. Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron)
   9.2 Buprofezin
   9.3 Cyromazine
*10. Inhibitoren der oxidativen Phosphorylierung, ATP-Disruptoren*
   10.1 Diafenthiuron
   10.2 Organotine (z.B. Azocyclotin, Cyhexatin, Fenbutatin-oxide)
*11. Entkoppler der oxidativen Phoshorylierung durch Unterbrechung des H-Protongradienten*
   11.1 Pyrrole (z.B. Chlorfenapyr)
   11.2 Dinitrophenole (z.B. Binapacyrl, Dinobuton, Dinocap, DNOC)
*12. Site-I-Elektronentransportinhibitoren*
   12.1 METI's (z.B. Fenazaquin, Fenpyroximate, Pyrimidifen, Pyridaben, Tebufenpyrad, Tolfenpyrad)
   12.2 Hydramethylnone
   12.3 Dicofol
*13. Site-II-Elektronentransportinhibitoren*
   13.1 Rotenone
*14. Site-III-Elektronentransportinhibitoren*
   14.1 Acequinocyl, Fluacrypyrim
*15. Mikrobielle Disruptoren der Insektendarmmembran*
   Bacillus thuringiensis-Stämme
*16. Inhibitoren der Fettsynthese*
   16.1 Tetronsäuren (z.B. Spirodiclofen, Spiromesifen)
   16.2 Tetramsäuren [z.B. 3-(2,5-Dimethylphenyl)-8-methoxy-2-oxo-1-azaspiro[4.5]dec-3-en-4-yl ethyl carbonate (alias: Carbonic acid, 3-(2,5-dimethylphenyl)-8-methoxy-2-oxo-1-azaspiro[4.5]dec-3-en-4-yl ethyl ester, CAS-Reg:-No.: 382608-10-8) and Carbonic acid, cis-3-(2,5-dimethylphenyl)-8-methoxy-2-oxo-1-azaspiro[4.5]dec-3-en-4-yl ethyl ester (CAS-Reg.-No.: 203313-25-1)]
*17. Carboxamide*
   (z.B. Flonicamid)
*18. Oktopaminerge Agonisten*
   (z.B. Amitraz)
*19. Inhibitoren der Magnesium-stimulierten ATPase*
   (z.B. Propargite)
*20. Agonisten des Ryanodin-Rezeptors,*
   20.1 Benzoesäuredicarboxamide [z.B. N²-[1,1-Dimethyl-2-(methylsulfonyl)ethyl]-3-iod-N¹-[2-methyl-4-[1,2,2,2-tetrafluor-1-(trifluonnethyl)ethyl]phenyl]-1,2-benzenedicarboxamide (CAS-Reg.-No.: 272451-65-7), Flubendiamide]
   20.2 Anthranilamide (z.B. DPX E2Y45 = 3-Brom-N-{4-chlor-2-methyl-6-[(methylamino)carbonyl]-phenyl}-1-(3-chlorpyridin-2-yl)-1H-pyrazol-5-carboxamid)
*21. Nereistoxin-Analoge*
   (z.B. Thiocyclam hydrogen oxalate, Thiosultap-sodium)
*22. Biologika, Hormone oder Pheromone*
   (z.B. Azadirachtin, Bacillus spec., Beauveria spec., Codlemone, Metarrhizium spec., Paecilomyces spec., Thuringiensin, Verticillium spec.)
*23. Wirkstoffe mit unbekannten oder nicht spezifischen Wirkmechanismen*
   23.1 Begasungsmittel (z.B. Aluminium phosphide, Methyl bromide, Sulfuryl fluoride)
   23.2 Selektive Fraßhemmer (z.B. Cryolite, Flonicamid, Pymetrozine)
   23.3 Milbenwachstumsinhibitoren (z.B. Clofentezine, Etoxazole, Hexythiazox)
   23.4 Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyrafluprole, Pyridalyl, Pyriprole, Sulfluramid, Tetradifon, Tetrasul, Triarathene, Verbutin, ferner die Verbindung 3-Methyl-phenyl-propylcarbamat (Tsumacide Z), die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO 96/37494, WO 98/25923), sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren, Safener bzw. Semiochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzeuteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, weiter entwickeltes Wurzelsystem, höhere Beständigkeit der Pflanzenart bzw. Pflanzensorte, gesteigertes Wachstum der Schösslinge, höhere Pflanzenvitalität, erhöhte Toleranz gegenüber hohen oder niedrigen Temperaturen, erhöhte Toleranz gegen Trockenheit oder gegen Wasser- bzw. Bodensalzgehalt, erhöhte Blühleiswng, erleichterte Ernte, Beschleunigung der Reife, höhere Ernteerträge, größere Früchte, höhere Pflanzengröße, grünere Blattfarbe, frühere Blüte, höhere Qualität und/oder höherer Ernährungswert der Ernteprodukte, höhere Zuckerkonzentration in den Früchten, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Trans") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Trait") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im Folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, z.B. Imidazolinonen, Sulfonylharnstoffen, Glyphosate oder Phosphinotricin (z.B. "PAT"-Gen). Die jeweils die gewünschten Eigenschaften ("Trans") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucoton® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Herstellung und die Verwendung der erfindungsgemäßen Wirkstoffe geht aus den folgenden Beispielen hervor.

### Herstellungsbeispiel

### Herstellung von Verbindung 9

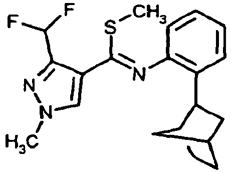

Zu einer Lösung bestehend aus 1.0 g (2.8 mmol) N-(2-Bicyclo[2.2.1]hept-2-ylphenyl)-3-(difluormethyl)-1-methyl-1H-pyrazol-4-thiocarboxamid in 10 ml Dimethylformamid werden bei 0°C 0.1 g (3.6 mmol) Natriumhydrid (80%ig) und 1.2g (8.3 mmol) Methyliodid in 5 ml Dimethylformamid zugegeben. Die Reaktionsmischung wird langsam auf Raumtemperatur erwärmt und für 1 Stunde bei dieser Temperatur gerührt. Zur Aufarbeitung wird die Reaktionsmischung auf Wasser gegossen und dreimal mit je 50 ml Ethylacetat extrahiert. Die vereinigten organischen Phasen werden mit gesättigter Kochsalzlösung gewaschen, über Natriumsulfat getrocknet und im Vakuum aufkonzentriert. Säulenchromatographie (Cyclohexan/Essigsäurethylester 4:1) liefert 0.7 g (1.9 mmol, 67 % der Theorie) Methyl N-(2-Bicyclo[2.2.1]hept-2-ylphenyl)-3-(difluormethyl)-1-methyl-1H-pyrazol-4-carbimidothioat [logP (pH 2.3) = 4.75].

Analog diesem Beispiel sowie entsprechend den allgemeinen Beschreibungen der erfindungsgemäßen Verfahren können die in der folgenden Tabelle 1 genannten Verbindungen der Formel (I) erhalten werden.

**Tabelle 1**

| | | | | | | |
|---|---|---|---|---|---|---|
| 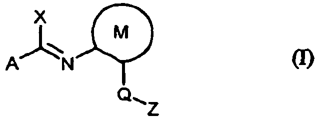 | | | | | | |

| **Nr.** | **X** | **M** | **Q** | **Z** | **A** | **LogP (pH 2,3)** |
|---|---|---|---|---|---|---|
| 1 | SO₂CH₃ | M-1, R⁷ = H | direkte Bindung | 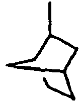 | 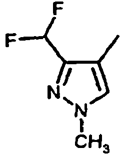 | |
| 2 | SCH₂CH(OCH₃)₂ | M-1, R⁷ = H | direkte Bindung | 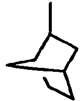 | 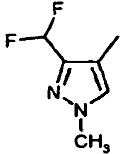 | |
| 3 | S(CH₂)₂OCH₃ | M-1, R⁷=H | direkte Bindung | 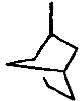 | 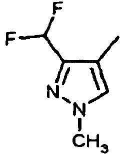 | 4,62 |
| 4 | S(CH₂)₃OH | M-1, R⁷ = H | direkte Bindung | 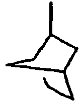 | 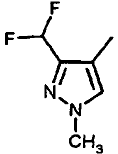 | |
| 5 | SCH₂C(O)NHCH₃ | M-1, R⁷=H | direkte Bindung | 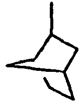 | 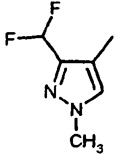 | |
| 6 | SCH₂CN | M-1, R⁷ = H | direkte Bindung | 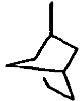 | 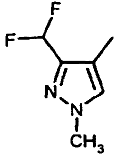 | 4,15 |
| 7 | SCH₂SCN | M-1, R⁷ = H | direkte Bindung | 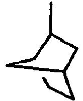 | 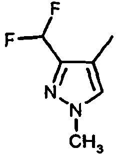 | 4,55 |
| 8 | SCH₂C≡CH | M-1, R⁷ = H | direkte Bindung | 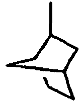 | 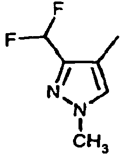 | 4,65 |
| 9 | SCH₃ | M-1, R⁷ = H | direkte Bindung | 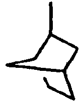 | 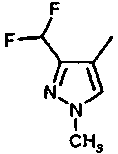 | 4,75 |
| 10 | S(CH₂)₂OH | M-1, R⁷ = H | direkte Bindung | 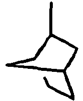 | 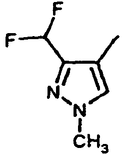 | 3,23 |
| 11 | SCH₂CO₂C₂H₅ | M-1, R⁷=H | direkte Bindung | 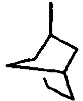 | 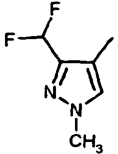 | 4,66 |
| 12 | 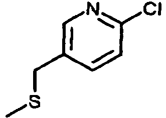 | M-1, R⁷ = H | direkte Bindung | 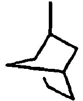 | 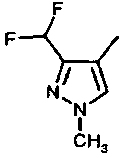 | 4,95 |
| 13 | S(CH₂)₁₁CH₃ | M-1, R⁷ = H | direkte Bindung | 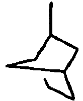 | 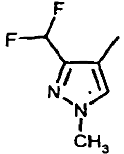 | 7,36 |
| 14 | 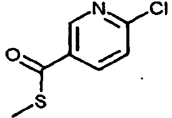 | M-1, R⁷= H | direkte Bindung | 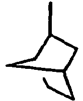 | 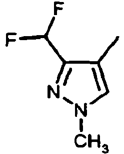 | |
| 15 | 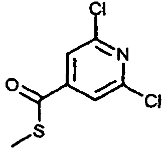 | M-1, R⁷ = H | direkte Bindung | 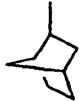 | 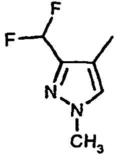 | |
| 16 | SC(O)CH₂OCH₃ | M-1, R⁷ = H | direkte Bindung | 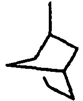 | 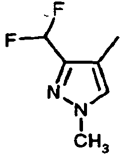 | |
| 17 | 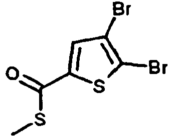 | M-1, R⁷ = H | direkte Bindung | 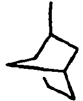 | 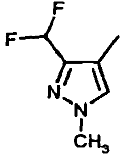 | |
| 18 | SC(O)C(CH₃)₃ | M-1, R⁷ = H | direkte Bindung | 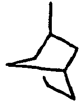 | 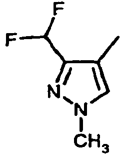 | 5,81 |
| 19 | 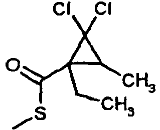 | M-1, R⁷=H | direkte Bindung | 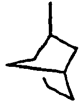 | 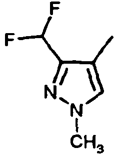 | 6,52 |
| 20 | 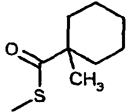 | M-1, R⁷=H | direkte Bindung | 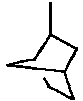 | 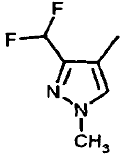 | 6,66 |

### Herstellung von Ausgangsstoffen der Formel (II-a)

### Herstellung von Verbindung (II-7)

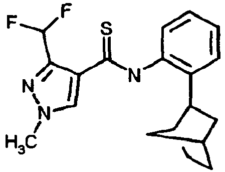

Zu einer Lösung bestehend aus 0.48 g (1.4 mmol) N-(2-Bicyclo[2.2.1]hept-2-ylphenyl)-3-(difluormethyl)-1-methyl-1H-pyrazol-4-carbonsäureamid in 20 ml Toluol werden 0.3g (1.4 mmol) Phosphorpentasulfid gegeben, die Reaktionsmischung wird für 2 Stunden bei 70°C gerührt. Zur Aufarbeitung wird die Reaktionsmischung auf Wasser gegossen, einmal mit 20 ml Toluol und zweimal mit je 20 ml Ethylacetat extrahiert. Die vereinigten organischen Phasen werden über Natriumsulfat getrocknet und im Vakuum aufkonzentriert. Säulenchromatographie (Gradient Cyclohexan/Essigsäureethylester) liefert 0.32g (0.89 mmol, 63% der Theorie) N-(2-Bicyclo[2.2.1]hept-2-ylphenyl)-3-(difluormethyl)-1-methyl-1H-pyrazol-4-thiocarboxamid [logP (pH 2.3)=3.66].

### Herstellung von Verbindung (II-8)

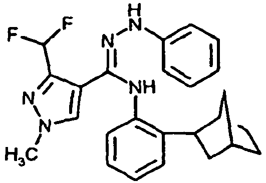

Zu einer Lösung bestehend aus 1.0 g (2.7 mmol) N-(2-Bicyclo[2.2.1]hept-2-ylphenyl)-3-(difluormethyl)-1-methyl-1H-pyrazol-4-thiocarboxamid in 10 ml Methanol werden 0.9 g (8.4 mmol) Phenylhydrazin in 10 ml Dimethylformamid gegeben. Die Reaktionsmischung wird für 16 Stunden bei 70°C gerührt. Zur Aufarbeitung wird die Reaktionsmischung auf Wasser gegossen und dreimal mit je 20 ml Dichlormethan extrahiert. Die vereinigten organischen Phasen werden über Natriumsulfat getrocknet und im Vakuum aufkonzentriert. Säulenchromatographie (Gradient Cyclohexan/Essigsäureethylester) liefert 0.42 g (0.96 mmol, 35 % der Theorie) an N-(2-Bicyclo[2.2.1]hept-2-ylphenyl)-3-(difluormethyl)-1-methyl-N'-phenyl-1H-pyrazol-4-carbohydrazonamid [logP (pH 2.3) = 3.66].

Analog diesem Beispiel sowie entsprechend den allgemeinen Beschreibungen der erfindungsgemäßen Verfahren können die in der folgenden Tabelle 2 genannten Verbindungen der Formel (III) erhalten werden.

**Tabelle 2**

| | | | | | | |
|---|---|---|---|---|---|---|
| 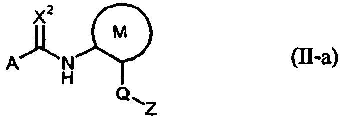 | | | | | | |

| **Nr.** | **X** | **M** | **Q** | **Z** | **A** | **LogP (pH 2,3)** |
|---|---|---|---|---|---|---|
| **II-1** | S | M-1, R⁷ = H | direkte Bindung | G5 | 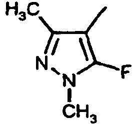 | 3,70 (bei pH 7.5) |
| **II-2** | S | M-1, R⁷ = 4-F | direkte Bindung | 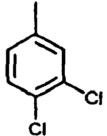 | 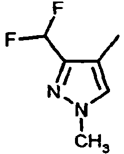 | |
| **II-3** | N-OCH₃ | M-1, R⁷ = H | direkte Bindung | 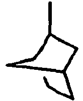 | 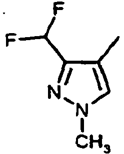 | |
| **II-4** | N-CH₃ | M-1, R⁷=H | direkte Bindung | 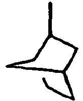 | 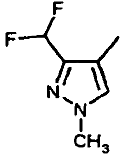 | |
| **II-5** | N-NH₂ | M-1, R⁷ = H | direkte Bindung | 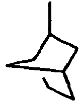 | 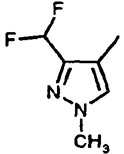 | |
| **II-6** | N-N(CH₃)₂ | M-1, R⁷ = H | direkte Bindung | 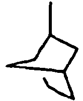 | 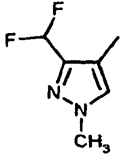 | |
| **II-7** | S | M-1, R⁷ = H | direkte Bindung | 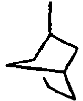 | 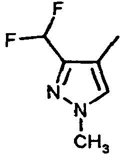 | 3,66 |
| **II-8** | 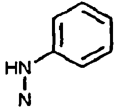 | M-1, R⁷ = H | direkte Bindung | 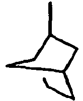 | 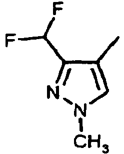 | 3,66 |
| **II-9** | N-OH | M-1, R⁷ = H | direkte Bindung | 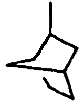 | 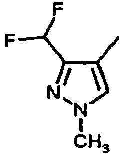 | 2,88 |

Die Bestimmung der in den voranstehenden Tabellen und Herstellungsbeispielen angegebenen logP-Werte erfolgt gemäß EEC-Directive 79/831 Annex V.A8 durch HPLC (High Performance Liquid Chromatography) an einer Phasenumkehrsäule (C 18). Temperatur: 43°C.
Die Bestimmung erfolgt im sauren Bereich bei pH 2.3 mit 0,1 % wässriger Phosphorsäure und Acetonitril als Eluenten; linearer Gradient von 10 % Acetonitril bis 90 % Acetonitril.
Die Bestimmung erfolgt im neutralen Bereich bei pH 7.5 mit 0,01-molarer wässriger Phosphatpuffer-Lösung und Acetonitril als Eluenten; linearer Gradient von 10 % Acetonitril bis 90 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanolen).
Die lambda-max-Werte wurden an Hand der W-Spektren von 200 nm bis 400 nm in den Maxima der chromatographischen Signale ermittelt.

### Anwendungsbeispiele

### Beispiel A

### Sphaerotheca - Test (Gurke) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.
Zur Prüfung auf protektive Wirksamkeit werden junge Pflanzen mit, der Wirkstoffzubereitung in der angegebenen Aufwandmenge besprüht. Nach Antrocknen des Spritzbelages werden die Pflanzen mit einer wässrigen Sporensuspension von *Sphaerotheca fuliginea* inokuliert. Die Pflanzen werden dann bei ca. 23°C und einer relativen Luftfeuchtigkeit von ca. 70 % im Gewächshaus aufgestellt. 7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle A**

| **Sphaerotheca - Test (Gurke) / protektiv** | | | |
|---|---|---|---|
| Wirkstoff Erfindungsgemäß | | Aufwandmenge an Wirkstoff in ppm | Wirkungsgrad in % |
| (10) | 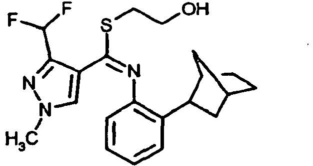 | 100 | 100 |
| (11) | 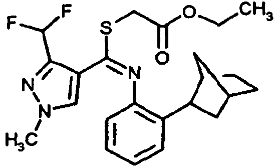 | 100 | 100 |
| (20) | 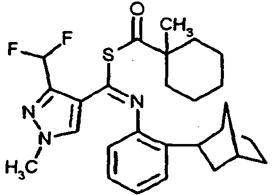 | 100 | 100 |

### Beispiel B

### Venturia - Test (Apfel) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers *Venturia inaequalis* inokuliert und verbleiben dann 1 Tag bei ca. 20°C und 100 % relativer Luftfeuchtigkeit in einer Inkubationskabine. Die Pflanzen werden dann im Gewächshaus bei ca. 21°C und einer relativen Luftfeuchtigkeit von ca. 90 % aufgestellt. 10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle B**

| **Venturia - Test (Apfel) / protektiv** | | | |
|---|---|---|---|
| Wirkstoff Erfindungsgemäß | | Aufwandmenge an Wirkstoff in ppm | Wirkungsgrad in % |
| (10) | 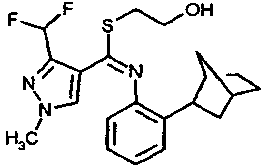 | 100 | 100 |
| (11) | 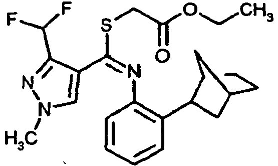 | 100 | 100 |
| (19) | 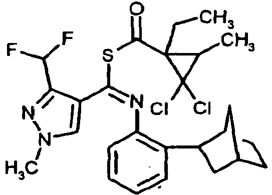 | 100 | 99 |
| (20) | 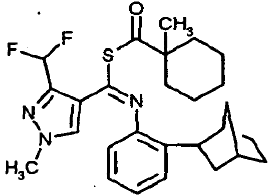 | 100 | 100 |

### Beispiel C

### Botrytis - Test (Bohne) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit *Botrytis cinerea* bewachsene Agarstückchen aufgelegt. Die inokulierten Pflanzen werden in einer abgedunkelten Kammer bei ca. 20°C und 100 % relativer Luftfeuchtigkeit aufgestellt. 2 Tage nach der Inokulation wird die Größe der Befallsflecken auf den Blättern ausgewertet. Dabei bedeutet 0% ein Wirkungsgrad, der demjenigen der Kontrolle entspricht, während ein Wirkungsgrad von 100 % bedeutet, dass kein Befall beobachtet wird.

**Tabelle C**

| **Botrytis - Test (Bohne) / protektiv** | | | |
|---|---|---|---|
| Wirkstoff Erfindungsgemäß | | Aufwandmenge an Wirkstoff in ppm | Wirkungsgrad in % |
| (10) | 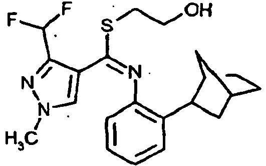 | 250 | 100 |
| (11) | 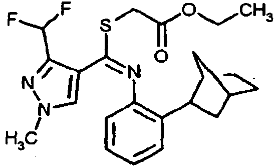 | 250 | 100 |

### Beispiel D

### Uromyces - Test (Bohne) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension des Bohnenrosterregers *Uromyces appendiculatus* inokuliert und verbleiben dann 1 Tag bei ca. 20°C. und 100 % relativer Luftfeuchtigkeit in einer Inkubationskabine. Die Pflanzen werden dann im Gewächshaus bei ca. 21°C und einer relativen Luftfeuchtigkeit von ca. 90 % aufgestellt. 10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle D**

| **Uromyces - Test (Bohne) / protektiv** | | | |
|---|---|---|---|
| Wirkstoff Erfindungsgemäß | | Aufwandmenge an Wirkstoff in ppm | Wirkungsgrad in % |
| (10) | 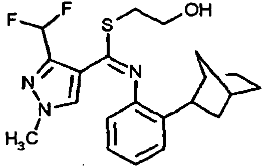 | 100 | 100 |
| (11) | 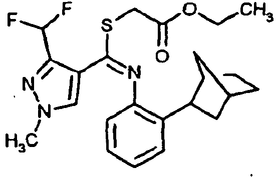 | 100 | 100 |
| (19) | 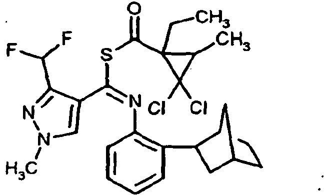 | 100 | 96 |
| (20) | 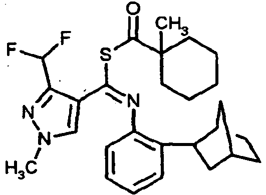 | 100 | 99 |

### Beispiel E

### Alternaria -Test (Tomate) / protektiv

| | | |
|---|---|---|
| Lösungsmittel: | 49 | Gewichtsteile N,N-Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Zur Prüfung auf protektive Wirksamkeit bespritzt man junge Tomatenpflanzen mit der Wirkstoffzubereitung in der angegebenen Aufwandmenge. 1 Tag nach der Behandlung werden die Pflanzen mit einer Sporensuspension von *Alternaria solani* inokuliert und stehen dann 24h bei 100 % relativer Feuchte und 20°C. Anschließend stehen die Pflanzen bei 96 % relativer Luftfeuchtigkeit und einer Temperatur von 20°C. 7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle E**

| **Alternaria -Test (Tomate) / protektiv** | | | |
|---|---|---|---|
| Wirkstoff Erfindungsgemäß | | Aufwandmenge an Wirkstoff in ppm | Wirkungsgrad in % |
| (10) | 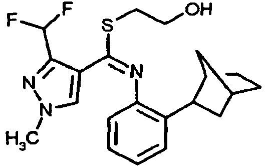 | 500 | 100 |
| (11) | 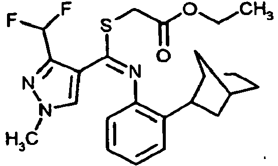 | 500 | 95 |
| (19) | 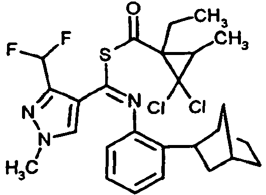 | 500 | 90 |
| (20) | 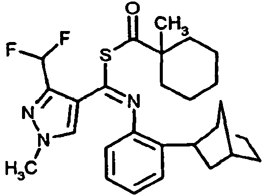 | 500 | 80 |

### Beispiel F

### Pyrenophora teres - Test (Gerste) / protektiv

| | | |
|---|---|---|
| Lösungsmittel: | 50 | Gewichtsteile N,N-Dimethylacetamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Zur Prüfung auf protektive Wirksamkeit werden junge Pflanzen mit der Wirkstoffzubereitung in der angegeben Aufwandmenge besprüht. Nach Antrocknen des Spritzbelages werden die Pflanzen mit einer Konidiensuspension von Pyrenophora teres besprüht. Die Pflanzen verbleiben 48 Stunden bei 20°C und 100 % relativer Luftfeuchtigkeit in einer Inkubationskabine. Die Pflanzen werden dann in einem Gewächshaus bei einer Temperatur von ca. 20°C und relativen Luftfeuchtigkeit von ca. 80 % aufgestellt. 8 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle F**

| **Pyrenophora teres - Test (Gerste) / protektiv** | | | |
|---|---|---|---|
| Wirkstoff Erfindungsgemäß | | Aufwandmenge an Wirkstoff in ppm | Wirkungsgrad in % |
| (11) | 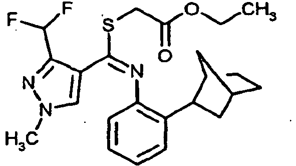 | 1000 | 100 |
| (19) | 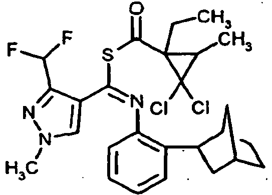 | 1000 | 92 |
| (20) | 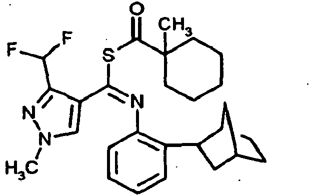 | 1000 | 100 |

### Beispiel G

### Puccinia - Test (Weizen) / protektiv

| | | |
|---|---|---|
| Lösungsmittel: | 50 | Gewichtsteile N,N-Dimethylacetamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von Puccinia recondita besprüht. Die Pflanzen verbleiben 48 Stunden bei 20°C und 100 % relativer Luftfeuchtigkeit in einer Inkubarionskabine. Die Pflanzen werden dann in einem Gewächshaus bei einer Temperatur von ca. 20°C und einer relativen Luftfeuchtigkeit von 80 % aufgestellt, um die Entwicklung von Rostpusteln zu begünstigen. 10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

**Tabelle G**

| **Puccinia - Test (Weizen) / protektiv** | | | |
|---|---|---|---|
| Wirkstoff Erfindungsgemäß | | Aufwandmenge an Wirkstoff in ppm | Wirkungsgrad in % |
| (11) | 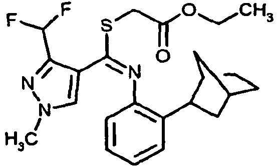 | 1000 | 100 |
| (19) | 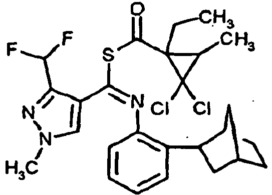 | 1000 | 93 |
| (20) | 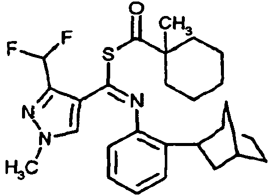 | 1000 | 100 |

## Patentansprüche

1. Carboxamide der Formel (I) 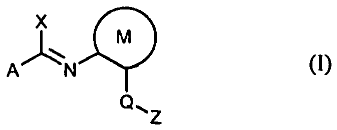 in welcher
X für OR¹, SR², NHR³, NR⁴R⁵, SOR⁶, SO₂R⁶ steht,
R¹ C₁-C₁₂-Alkyl, C₂-C₈-Alkenyl, C₂-C₈-Alkinyl, C₁-C₈-Halogenalkyl, Hydroxy-C₁-C₈-alkyl, (C₁-C₄-Alkoxy)-C₁-C₈-alkyl, Bis(C₁-C₄-alkoxy)-C₁-C₈-alkyl, (C₁-C₄-Alkoxy)-carbonyl-(C₁-C₄-alkyl); jeweils gegebenenfalls durch Halogen, C₁-C₄-Alkyl oder C₁-C₄-Halogenalkyl substituiertes Phenyl-(C₁-C₂-alkyl) oder Hetaryl-(C₁-C₂-alkyl); jeweils gegebenenfalls durch C₁-C₄-Alkoxy, Halogen, C₁-C₄-Alkyl oder C₁-C₄-Halogenalkyl substituiertes (C₁-C₆-Alkyl)carbonyl, (C₃-C₈-Cycloalkyl)carbonyl, Phenylcarbonyl oder Hetarylcarbonyl,
R² C₁-C₁₂-Alkyl, C₂-C₈-Alkenyl, C₂-C₈-Alkinyl, C₁-C₈-Halogenalkyl, Hydroxy-C₁-C₈-alkyl, (C₁-C₄-Alkoxy)-C₁-C₈-alkyl, Bis(C₁-C₄-alkoxy)-C₁-C₈-alkyl, Cyano-(C₁-C₄-alkyl), -CH₂S-CN, (C₁-C₄-Alkoxy)-carbonyl-(C₁-C₄-alkyl); jeweils gegebenenfalls durch Halogen, C₁-C₄-Alkyl oder C₁-C₄-Halogenalkyl substituiertes Phenyl-(C₁-C₂-alkyl) oder Hetaryl-(C₁-C₂-alkyl); jeweils gegebenenfalls durch C₁-C₄-Alkoxy, Halogen, C₁-C₄-Alkyl oder C₁-C₄-Halogenalkyl substituiertes (C₁-C₆-Alkyl)carbonyl, (C₃-C₈-Cycloalkyl)carbonyl, Phenylcarbonyl oder Hetarylcarbonyl,
R³, R⁴ und R⁵ unabhängig voneinander für Amino, Hydroxy, Cyano, C₁-C₁₂-Alkyl, C₁-C₈-Alkoxy, C₂-C₈-Alkenyl, C₂-C₈-Alkinyl, C₁-C₆-Alkylamino, Di(C₁-C₆-alkyl)amino, Hydroxy-C₁-C₈-alkyl, (C₁-C₄-Alkoxy)-C₁-C₈-alkyl, Bis(C₁-C₄-alkoxy)-C₁-C₈-alkyl, Cyano-(C₁-C₄-alkyl), Phenoxy, Phenylamino, Benzyloxy oder Benzylamino stehen,
R⁶ C₁-C₁₂-Alkyl oder C₁-C₈-Halogenalkyl steht,
M für einen jeweils einfach durch R⁷ substituierten Phenyl-, Thiophen-, Pyridin-, Pyrimidin-, Pyridazin oder Pyrazin-Ring oder für einen durch R⁸ substituierten Thiazol-Ring steht,
R⁷ für Wasserstoff, Fluor, Chlor, Methyl, iso-Propyl, Methylthio oder Trifluormethyl steht,
R⁸ für Wasserstoff, Methyl, Methylthio oder Trifluormethyl steht,
Q für eine direkte Bindung, C₁-C₄-Alkylen, C₂-C₄-Alkenylen, O, S, SO, SO₂ oder NR⁹ steht,
R⁹ für Wasserstoff, C₁-C₈-Alkyl, C₁-C₄-Alkoxy-C₁-C₄-alkyl, C₁-C₄-Alkylthio-C₁-C₄-alkyl, C₂-C₈-Alkenyl, C₂-C₈-Alkinyl, C₁-C₆-Halogenalkyl, C₂-C₆-Halogenalkenyl, C₂-C₆-Halogenalkinyl oder C₃-C₆-Cycloalkyl steht,
Z für Z¹, Z², Z³, Z⁴, Z⁵ oder Z⁶ steht,
Z¹ für gegebenenfalls einfach bis fünffach, gleich oder verschieden substituiertes Phenyl steht,
Z² für gegebenenfalls einfach bis dreifach, gleich oder verschieden substituiertes Pyridinyl steht,
Z³ für jeweils gegebenenfalls einfach oder mehrfach, gleich oder verschieden durch Halogen, Alkyl und/oder -(CR¹⁰R¹¹)ₘSiR¹²R¹³R¹⁴ substituiertes Cycloalkyl oder Bicycloalkyl steht,
Z⁴ für unsubstituiertes C₂-C₂₀-Alkyl oder für einfach oder mehrfach, gleich oder verschieden durch Halogen, Alkylthio, Alkylsulfinyl, Alkylsulfonyl, Alkoxy, Alkylamino, Dialkylamino, Halogenalkylthio, Halogenalkylsulfinyl, Halogenalkylsulfonyl, Halogenalkoxy, Halogenalkylamino, Halogen-dialkylamino, -SiR¹²R¹³R¹⁴ und/oder C₃-C₆-Cycloalkyl substituiertes C₁-C₂₀-Alkyl steht, wobei der Cycloalkylteil seinerseits gegebenenfalls einfach oder mehrfach, gleich oder verschieden durch Halogen und/oder C₁-C₄-Alkyl substituiert sein kann,
Z⁵ für jeweils gegebenenfalls einfach oder mehrfach, gleich oder verschieden durch Halogen, Alkylthio, Alkylsulfinyl, Alkylsulfonyl, Alkoxy, Alkylamino, Dialkylamino, Halogenalkylthio, Halogenalkylsulfinyl, Halogenalkylsulfonyl, Halogenalkoxy, Halogenalkylamino, Halogen-dialkylamino, -SiR¹²R¹³R¹⁴ und/oder C₃-C₆-Cycloalkyl substituiertes C₂-C₂₀-Alkenyl oder C₂₀-C₂₀-Alkinyl steht, wobei der Cycloalkylteil seinerseits gegebenenfalls einfach oder mehrfach, gleich oder verschieden durch Halogen und/oder C₁-C₄-Alkyl substituiert sein kann,
Z⁶ für einen gegebenenfalls einfach oder mehrfach substituierten, gesättigten oder ungesättigten 3- bis 7-gliedrigen Ring steht, welcher ein Siliziumatom als Ringglied enthält, wobei dann Q für eine direkte Bindung oder C₁-C₄-Alkylen steht,
R¹⁰ für Wasserstoff oder C₁-C₄-Alkyl steht,
R¹¹ für Wasserstoff oder C₁-C₄-Alkyl steht,
m für 0, 1, 2 oder 3 steht,
R¹² und R¹³ unabhängig voneinander für Wasserstoff, C₁-C₈-Alkyl, C₁-C₈-Alkoxy, C₁-C₄-Alkoxy-C₁-C₄-alkyl, C₁-C₄-Alkylthio-C₁-C₄-alkyl oder C₁-C₆-Halogenalkyl stehen,
R¹⁴ für Wasserstoff, C₁-C₈-Alkyl, C₁-C₈-Alkoxy, C₁-C₄-Alkoxy-C₁-C₄-alkyl, C₁-C₄-Alkylthio-C₁-C₄-alkyl, C₂-C₈-Alkenyl, C₂-C₈-Alkinyl, C₁-C₆-Halogenalkyl, C₂-C₆-Halogenalkenyl, C₂-C₆-Halogenalkinyl, C₃-C₆-Cycloalkyl, oder für jeweils gegebenenfalls substituiertes Phenyl oder Phenylalkyl steht,
oder
M-Q-Z gemeinsam für jeweils gegebenenfalls einfach bis dreifach durch Methyl substituiertes 1H-2,3-Dihydro-inden-4-yl, 1,3-Dihydro-2-benzofuran-4-yl oder 1,3-Dihydro-2-benzothien-4-yl stehen,
A für A1 steht 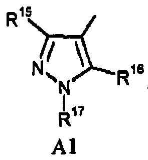
R¹⁵ für Wasserstoff, Cyano, Halogen, Nitro, C₁-C₄-Alkyl, C₁-C₄-Alkoxy C₁-C₄-Alkylthio, C₃-C₆-Cycloalkyl, C₁-C₄-Halogenalkyl, C₁-C₄-Halogenalkoxy oder C₁-C₄-Halogenalkylthio mit jeweils 1 bis 5 Halogenatomen, Aminocarbonyl oder Aminooarbonyl-C₁-C₄-alkyl steht,
R¹⁶ für Wasserstoff, Halogen, Cyano, C₁-C₄-Alkyl, C₁-C₄-Alkoxy oder C₁-C₄-Alkylthio steht,
R¹⁷ für Wasserstoff, C₁-C₄-Alkyl, Hydroxy-C₁-C₄-alkyl, C₂-C₆-Alkenyl, C₃-C₆-Cycloalkyl, C₁-C₄-Alkylthio-C₁-C₄-alkyl, C₁-C₄-Alkoxy-C₁-C₄-alkyl, C₁-C₄-Halogenalkyl, C₁-C₄-Hälogenalkylthio-C₁C₄-alkyl, C₁-C₄-Halogenalkoxy-C₁-C₄-alkyl mit jeweils 1 bis 5 Halogenatomen, oder Phenyl steht,

2. Carboxamide gemäß Anspruch 1 der Formeln (I-a), (I-b), (I-e), (I-f) oder (I-d) 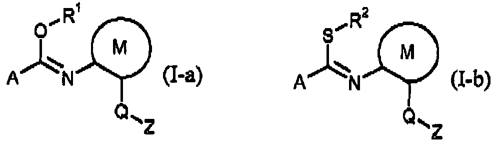 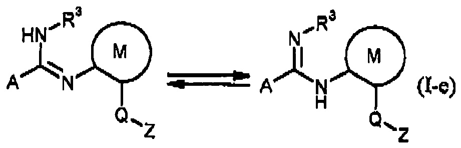 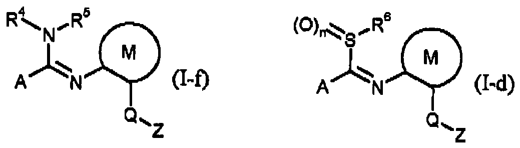 in welchen
R¹, R², R³, R⁴, R⁵, R⁶, M, Q, Z und A die in Anspruch 1 angegebenen Bedeutungen haben,
n für 1 oder 2 steht.

3. Verfahren zum Herstellen von Carboxamiden der Formel (I) gemäß Anspruch 1, **dadurch gekennzeichnet**,
(a) für den Fall, dass X für OR¹ steht, dass man Carboxamide der Formel (II-a) 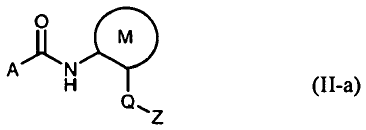 in welcher M, Q, Z und A die in Anspruch 1 angegebenen Bedeutungen haben, gegebenenfalls in Gegenwart einer Base mit einem Alkylierungsmittel der Formel (III-a)
LG¹—R¹ (III-a)
in welcher
R¹ die in Anspruch 1 angegebenen Bedeutungen hat und
LG¹ für eine Abgangsgruppe steht,
oder mit einem Meerweinsalz der Formel (III-d)
(R¹)₃O⁺E⁻ (III-d)
in welcher
R¹ die in Anspruch 1 angegebenen Bedeutungen hat und
E für BF₄, SbF₆ oder SbCl₆ steht,
umsetzt und Carboxamide der Formel (I-a) 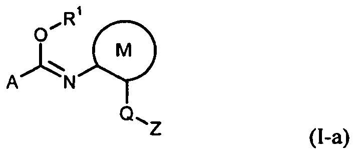 erhält, in welcher R¹, M, Q, Z und A die in Anspruch 1 angegebenen Bedeutungen haben,
(b) für den Fall, dass X für SR², SOR⁶ oder SO₂R⁶ steht, dass man in einem ersten Schritt Carboxamide der Formel (II-a) 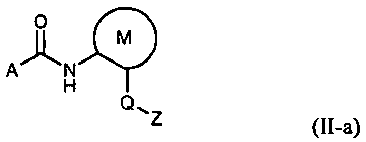 in welcher M, Q, Z und A die in Anspruch 1 angegebenen Bedeutungen haben, gegebenenfalls in Gegenwart eines Verdünnungsmittels mit einem Thionierungsmittel umsetzt
und so Carboxamide der Formel (II-b) 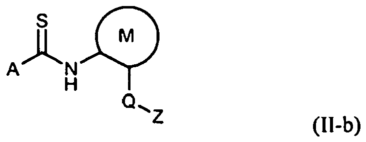 erhält, in welcher M, Q, Z und A die in Anspruch 1 angegebenen Bedeutungen haben, weiche man in einem zweiten Schritt gegebenenfalls in Gegenwart einer Base mit einem Alkylierungsmittel der Formel (III-b) oder der Formel (III-c)
LG²―R² (III-b)
LG⁶―R⁶ (III-c)
in welchen
R² und R⁶ die in Anspruch 1 angegebenen Bedeutungen haben,
LG² für eine Abgangsgruppe steht,
LG⁶ für eine Abgangsgruppe steht,
umsetzt und Carboxamide der Formel (I-b) oder der Formel (I-c) 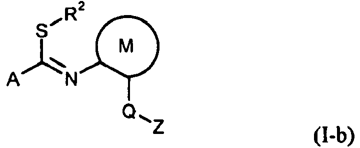 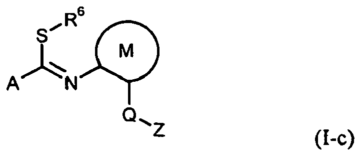 erhält, in welchen R², R⁶, M, Q, Z und A die in Anspruch 1 angegebenen Bedeutungen haben,
und in einem dritten Schritt die Carboxamide der Formel (I-c) anschließend mit einem Oxidationsmittel gegebenenfalls in Gegenwart eines Verdünnungsmittels, gegebenenfalls in Gegenwart eines Säurebindemittels und gegebenenfalls in Gegenwart eines Katalysators umsetzt und so die Carboxamide der Formel (I-d) 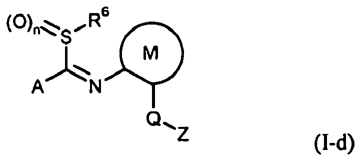 erhält, in welcher
R⁶ M, Q, Z und A die in Anspruch 1 angegebenen Bedeutungen haben und
n für 1 oder 2 steht,
(c) für den Fall, dass X für NHR³ oder NR⁴R⁵ steht, dass man Carboxamide der Formel (II-b) 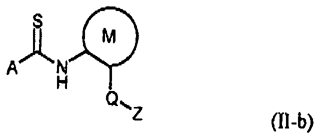 in welcher M, Q, Z und A die in Anspruch 1 angegebenen Bedeutungen haben,
gegebenenfalls in Gegenwart einer Base mit einem Amidierungsmittel der Formel (IV-a) oder der Formel (IV-b) 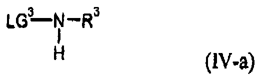 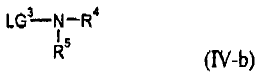 in welchen
R³, R⁴ und R⁵ die in Anspruch 1 angegebenen Bedeutungen haben und
LG³ für eine Abgangsgruppe steht,
umsetzt und Carboxamide der (I-e) oder der Formel (I-f) 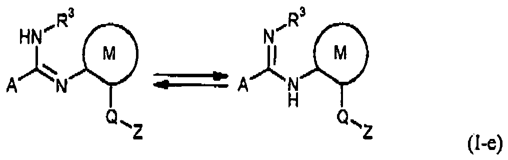 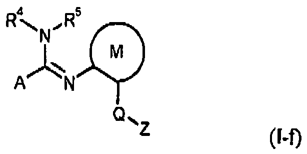 erhält, in welchen R³, R⁴, R⁵, M, Q, Z und A die in Anspruch 1 angegebenen Bedeutungen haben.

4. Mittel zum Bekämpfen unerwünschter Mikroorganismen, go**kennzeichnet durch** einen Gehalt an mindestens einem Carboxamid der Formel (I) gemäß Anspruch 1 neben Streckmitteln und/oder oberflächenaktiven Stoffen.

5. Verwendung von Carboxamiden der Formel (I) gemäß Anspruch 1 zum Bekämpfen unerwünschter Mikroorganismen.

6. Verfahren zum Bekämpfen unerwünschter Mikroorganismen, **dadurch gekennzeichnet, dass** man Carboxamide der Formel (I) gemäß Anspruch 1 auf die Mikroorganismen und/oder deren Lebensraum aufbringt

7. Verfahren zum Herstellen von Mitteln zum Bekämpfen unerwünschter Mikroorganismen, **dadurch gekennzeichnet, dass** man Carboxamide der Formel (I) gemäß Anspruch 1 mit Streckmitteln und/oder oberflächenaktiven Stoffen vermischt.

## Claims

1. Carboxamides of the formula (I) 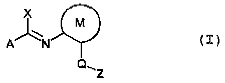 in which
X represents OR¹, SR², NHR³, NR⁴R⁵, SOR⁶, SO₂R⁶,
R¹ represents C₁-C₁₂-alkyl, C₂-C₈-alkenyl, C₂-C₈-alkynyl, C₁-C₈-haloalkyl, hydroxy-C₁-C₈-alkyl, (C₁-C₄-alkoxy)-C₁-C₈-alkyl, bis(C₁-C₄-alkoxy)-C₁-C₈-alkyl, (C₁-C₄-alkoxy)carbonyl-(C₁-C₄-alkyl); in each case optionally halogen-, C₁-C₄-alkyl- or C₁-C₄-haloalkyl-substituted phenyl-(C₁-C₇-alkyl) or hetaryl-(C₁-C₂-alkyl); in each case optionally C₁-C₄-alkoxy-, halogen-, C₁-C₄-alkyl- or C₁-C₄-haloalkyl-substituted (C₁-C₆-alkyl)carbonyl, (C₃-C₈-cycloalkyl)carbonyl, phenylcarbonyl or hetarycarbonyl,
R² represents C₁-C₁₂-alkyl, C₂-C₈-alkenyl, C₂-C₈-alkynyl, C₁-C₈-haloalkyl, hydroxy-C₁-C₈-alkyl, (C₁-C₄-alkoxy)-C₁-C₈-alkyl, bis(C₁-C₄-alkoxy)-C₁-C₈-alkyl, cyano-(C₁-C₄-alkyl), -CH₂S-CN, (C₁-C₄-alkoxy)carbonyl-(C₁-C₄-alkyl); in each case optionally halogen-, C₁-C₄-alkyl- or C₁-C₄-haloalkyl-substituted phenyl-(C₁-C₂-alkyl) or hetaryl-(C₁-C₂-alkyl); in each case optionally C₁-C₄-alkoxy-, halogen-, C₁-C₄-alkyl- or C₁-C₄-haloalkyl-substituted (C₁-C₆-alkyl) carbonyl, (C₃-C₈-cycloalkyl) carbonyl, Phenylcarbonyl or hetarylcarbonyl,
R³, R⁴ and R⁵ independently of one another represent amino, hydroxy, cyano, C₁-C₁₂-alkyl, C₁-C₈-alkoxy, C₂-C₈-alkenyl, C₂-C₈-alkynyl, C₁-C₆-alkylamino, di(C₁-C₆-alkyl)amino, bydroxy-C₁-C₈-alkyl, (C₁-C₅-alkoxy)-C₁-C₈-alkyl, bis(C₁-C₄-alkoxy)-C₁-C₈-alkyl, cyano-(C₁-C₄-alkyl), phenoxy, phenylamino, benzyloxy or benzylamino,
R⁶ represents C₁-C₁₂-alkyl or C₁-C₈-haloalkyl,
M represents a phenyl, thiophene, pyridine, pyrimidine, pyridazine or pyrazine ring, each of which is monosubstituted by R⁷, or represents a thiazole ring which is substituted by R⁸,
R⁷ represents hydrogen, fluorine, chlorine, methyl, isopropyl, methylthio or trifluoromethyl,
R⁸ represents hydrogen, methyl, methylthio or trifluoromethyl,
Q represents a direct bond, C₁-C₄-alkylene, C₂-C₄-alkenylene, O, S, SO, SO₂ or NR⁹,
R⁹ represents hydrogen, C₁-C₈-alkyl, C₁-C₄-alkoxy-C₁-C₄-alkyl, C₁-C₄-alkylthio-C₂-C₄-alkyl, C₂-C₈-alkenyl, C₂-C₈-alkynyl, C₁-C₅-haloalkyl, C₂-C₆-haloalkenyl, C₂-C₆-haloalkynyl or C₃-C₆-cycloalkyl,
Z represents Z¹, Z², Z³, Z⁴, Z⁵ or Z⁶,
Z¹ represents phenyl which is optionally mono- to pentasubstituted by identical or different substituents,
Z² represents pyridinyl which is optionally mono-to trisubstituted by identical or different substituents,
Z³ represents cycloalkyl or bicycloalkyl, each of which is optionally mono- or polysubstituted by identical or different halogen, alkyl and/or -(CR¹⁰R¹¹)ₘSiR¹²R¹³R¹⁴ substituents,
Z⁴ represents unsubstituted C₂-C₂₀-alkyl or represents C₁-C₂₀-alkyl which is mono- or polysubstituted by identical or different halogen, alkylthio, alkylsulphinyl, alkylsulphonyl, alkoxy, alkylamino, dialkylamino, haloalkylthio, haloalkylsulphinyl, haloalkylsulphonyl, haloalkoxy, haloalkylamino, halodialkylamino, -SiR¹²R¹³R¹⁴ and/or C₃-C₆-cycloalkyl susbstituents, where the cycloalkyl moiety for its part may optionally be mono- or polysubstituted by identical or different halogen and/or C₁-C₄-alkyl substituents,
Z⁵ represents C₂-C₂₀-alkenyl or C₂-C₂₀-alkynyl, each of which is optionally mono- or polysubstituted by identical or different halogen, alkylthio, alkylsulphinyl, alkylsulphonyl, alkoxy, alkylamino, dialkylamino, haloalkylthio, haloalkylsulphinyl, haloalkylsulphonyl, haloalkoxy, haloalkylamino, halodialkylamino, -SiR¹²R¹³R¹⁴ and/or C₃-C₆-cycloalkyl substituents, where the cycloalkyl moiety for its part may optionally be mono- or polysubstituted by identical or different halogen and/or C₁-C₄-alkyl substituents,
Z⁶ represents an optionally mono- or polysubstituted saturated or unsaturated 3-to 7-membered ring which contains a silicon atom as ring member, in which case Q represents a direct bond or C₁-C₄-alkylene,
R¹⁰ represents hydrogen or C₁-C₄-alkyl,
R¹¹ represents hydrogen or C₁-C₄-alkyl,
m represents 0, 1, 2 or 3,
R¹² and R¹³ independently of one another represent hydrogen, C₁-C₈-alkyl, C₁-C₈-alkoxy, C₁-C₄-alkoxy-C₁-C₄-alkyl, C₁-C₄-alkylthio-C₁-C₄-alkyl or C₁-C₆-haloalkyl,
R¹⁴ represents hydrogen, C₁-C₈-alkyl, C₁-C₈-alkoxy, C₁-C₄-alkoxy-C₁-C₄-alkyl, C₁-C₄-alkylthio-C₁-C₄-alkyl, C₂-C₈-alkenyl, C₂-C₈-alkynyl, C₁-C₆-haloalkyl, C₂-C₆-haloalkenyl, C₂-C₆-haloalkynyl, C₃-C₆-cycloalkyl, or represents in each case optionally substituted phenyl or phenylalkyl,
or
M-Q-Z together represent 1H-2,3-dihydroinden-4-yl, 1,3-dihydro-2-benzofuran-4-yl or 1,3-dihydro-2-benzothien-4-yl, each of which is optionally mono- to trisubstituted by methyl,
A represents A1
R¹⁵ represents hydrogen, cyano, halogen, nitro, C₁-C₄-alkyl, C₁-C₄-alkoxy, C₁-C₄-alkylthio, C₃-C₆-cycloalkyl, C₁-C₄-haloalkyl, C₁-C₄-haloalkoxy or C₁-C₄-haloalkylthio having in each case 1 to 5 halogen atoms, aminocarbonyl or aminocarbonyl-C₁-C₄-alkyl,
R¹⁶ represents hydrogen, halogen, cyano, C₁-C₄-alkyl, C₁-C₄-alkoxy or C₁-C₄-alkylthio,
R¹⁷ represents hydrogen, C₁-C₄-alkyl, hydroxy-C₁-C₄-alkyl, C₂-C₆-alkenyl, C₃-C₆-cycloalkyl, C₁-C₄-alkylthio-C₁-C₄-alkyl, C₁-C₄-alkoxy-C₁-C₄-alkyl, C₁-C₄-haloalkyl, C₁-C₄-haloalkyltbio-C₁-C₄-alkyl, C₁-C₄-haloalkoxy-C₁-C₄-alkyl having in each case 1 to 5 halogen atoms, or phenyl.

2. Carboxamides according to Claim 1 of the formulae (I-a), (I-b), (I-e), (I-f) and (I-d) 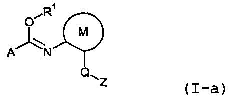 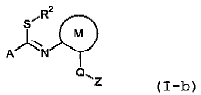 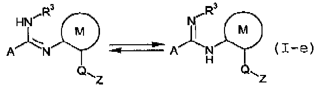 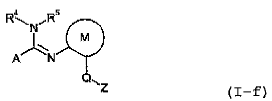 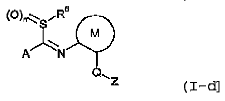 in which
R¹, R², R³, R⁴, R⁵, R⁶, M, Q, Z and A are as defined in Claim 1,
n represents 1 or 2.

3. Process for preparing carboxamides of the formula. (I) according to Claim 1, **characterized in that**
(a) if X represents OR¹, carboxamides of the formula (II-a) 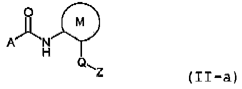 in which M, Q, Z and A are as defined in Claim 1
are, if appropriate, in the presence of a base, reacted with an alkylating agent of the formula (III-a)
LG¹―R¹ (III-a)
in which
R¹ is as defined in Claim 1 and
LG¹ represents a leaving group
or with a Meerwein salt of the formula (III-d)
(R¹)₃O⁺E⁻ (III-d)
in which
R¹ is as defined in Claim 1 and
E represents BF₄, SbF₆ or SbCl₆,
giving carboxamides of the formula (I-a) 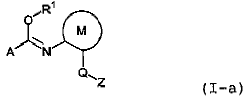 in which R¹, M, Q, Z and A are as defined in Claim 1,
(b) if X represents SR², SOR⁶ or SO₂R⁶, in a first step carboxamides of the formula (II-a) 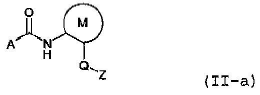 in which M, Q, Z and A are as defined in Claim 1
are, if appropriate in the presence of a diluent, reacted with a thionating agent,
thus giving carboxamides of the formula (II-b) 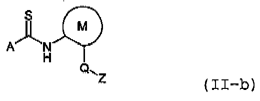 in which M, Q, Z and A are as defined in Claim 1
which, in a second step, are, if appropriate in the presence of a base, reacted with an alkylating agent of the formula (III-b) or the formula (III-c)
LG²―R² (III-b)
LG⁸―R⁸ (III-c)
in which
R² and R⁶ are as defined in Claim 1,
LG² represents a leaving group,
LG⁶ represents a leaving group,
giving carboxamides of the formula (I-b) or the formula (I-c) 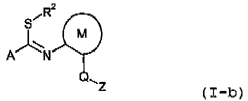 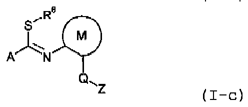 in which R², R⁶, M, Q, Z and A are as defined in Claim 1,
and, in a third step, the carboxamides of the formula (I-c) are then reacted with an oxidizing agent, if appropriate in the presence of a diluent, if appropriate in the presence of an acid binder and if appropriate in the presence of a catalyst, thus giving the carboxamides of the formula (I-d) 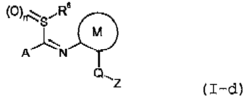 in which
R⁶, M, Q, Z and A are as defined in Claim 1 and
n represents 1 or 2,
(c) if X represents NHR³ or NR⁴R⁵, carboxamides of the formula (II-b) 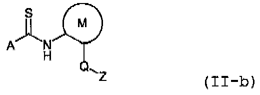 in which M, Q, Z and A are as defined in Claim 1
are, if appropriate in the presence of a base, reacted with an amidating agent of the formula (IV-a) or the formula (IV-b) 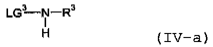 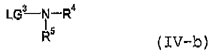 in which
R³, R⁴ and R⁵ are as defined in Claim 1 and
LG³ represents a leaving group,
giving carboxamides of the formula (I-e) or the formula (I-f) 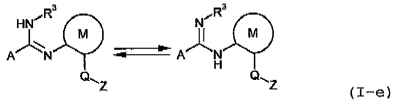 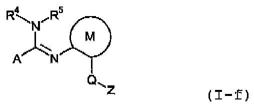 in which R³, R⁴, R⁵, M, Q, Z and A are as defined in Claim 1.

4. Compositions for controlling unwanted microorganisms, **characterized in that** they comprise at least one carboxamide of the formula (I) according to Claim 1, in addition to extenders and/or surfactants.

5. Use of carboxamides of the formula (I) according to Claim 1 for controlling unwanted microorganisms.

6. Method for controlling unwanted microorganisms, **characterized in that** carboxamides of the formula (I) according to Claim 1 are applied to the microorganisms and/or their habitat.

7. Process for preparing compositions for controlling unwanted microorganisms, **characterized in that** carboxamides of the formula (I) according to Claim 1 are mixed with extenders and/or surfactants.

## Revendications

1. Carboxamides de formule (I) 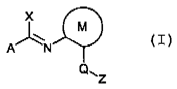 dans laquelle
X représente OR¹, SR², NHR³, NR⁴R⁵, SOR⁶, SO₂R⁶,
R¹ représente un groupe alkyle en C₁-C₁₂, alcényle en C₂-C₈, alcynyle en C₂-C₈, halogénoalkyle en C₁-C₈, hydroxyalkyle en C₁-C₈, alcoxy(C₁-C₄)-alkyle(C₁-C₈), bis[ alcoxy(C₁-C₄)] -alkyle(C₁-C₈), alcoxy(C₁-C₄)-carbonyl-alkyle(C₁-C₄) ; un groupe phényl-alkyle(C₁-C₂) ou hétéroaryl-alkyle(C₁-C₂) chacun éventuellement substitué par halogène, alkyle en C₁-C₄ ou halogénoalkyle en C₁-C₄ ; un groupe alkyl(C₁-C₆)carbonyle, cycloalkyl(C₃-C₈)carbonyle, phénylcarbonyle ou hétéroarylcarbonyle chacun éventuellement substitué par alcoxy en C₁-C₄, halogène, alkyle en C₁-C₄ ou halogénoalkyle en C₁-C₄ ;
R² représente un groupe alkyle en C₁-C₁₂, alcényle en C₂-C₈, alcynyle en C₂-C₈, halogénoalkyle en C₁-C₈, hydroxyalkyle en C1-C₈, alcoxy(C₁-C₄)-alkyle (C₁-C₈), bis[alcoxy(C₁-C₄)] -alkyle (C₁-C₈), cyano-alkyle (C₁-C₄), -CH₂S-CN, alcoxy(C₁-C₄)-carbonyl-alkyle(C₁-C₄) ;un groupe phénylalkyle(C₁-C₂) ou hétéroaxyl-alkyle(C₁-C₂) chacun éventuellement substitué par halogène, alkyle en C₁-C₄ ou halogénoalkyle en C₁-C₄ ; un groupe alkyl(C₁-C₆)carbonyle, cycloalkyl(C₃-C₈)carbonyle, phénylcarbonyle ou hétéroarylcarbonyle chacun éventuellement substitué par alcoxy en C₁-C₄, halogène, alkyle en C₁-C₄ ou halogénoalkyle en C₁-C₄ ;
R³, R⁴ et R⁵ représentent, chacun indépendamment, un groupe amino, hydroxy, cyano, alkyle en C₁-C₁₂, alcoxy en C₁-C₈, alcényle en C₂-C₈, alcynyle en C₂-C₈, alkyl(C₁-C₆)amino, di[alkyl(C₁-C₆)]amino, hydroxyalkyle(C₁-C₈), alcoxy(C₁-C₄)-alkyle(C₁-C₈), bis[alcoxy(C₁-C₄)] -alkyle(C₁-C₈), cyano-alkyle (C₁-C₄), phénoxy, phénylamino, benzyloxy ou benzylamino,
R⁶ représente un groupe alkyle en C₁-C₁₂ ou halogénoalkyle en C₁-C₈,
M représente un cycle phényle, thiophène, pyridine, pyrimidine, pyridazine ou pyrazine, chacun monosubstitué par R⁷, ou représente un cycle thiazole substitué par R⁸,
R⁷ représente un atome d'hydzogène, de fluor, de chlore, un groupe méthyle, isopropyle, méthylthio ou trifluorométhyle,
R⁸ représente un atome d'hydrogène, un groupe méthyle, méthylthio ou trifluorométhyle,
Q représente une liaison directe, un groupe alkyléne en C₁-C₄, alcénylène en C₂-C₄, O, S, SO, SO₂ ou NR⁹,
R⁹ représente un atome d'hydrogène, un groupe alkyle en C₁-C₈, alcoxy(C₁-C₄)-alkyle(C₁-C₄), alkyl(C₁-C₄)thio-alkyle(C₁-C₄), alcényle en C₂-C₈, alcynyle en C₂-C₈, halogénoalkyle en C₁-C₆, halogénoalcényle en C₂-C₆, halogénoalcynyle en C₂-C₆ ou cycloalkyle en C₃-C₆,
Z représente Z¹, Z², Z³, Z⁴, Z⁵ ou Z⁶,
Z¹ représente un groupe phényle portant éventuellement un à cinq substituants identiques ou différents,
Z² représente un groupe pyridinyle portant éventuellement un à trois substituants identiques ou différents,
Z³ représente un groupe cycloalkyle ou bicycloalkyle portant chacun éventuellement un ou plusieurs substituants, identiques ou différents, halogène, alkyle et/ou (CR¹⁰R¹¹)ₘSiR¹²R¹³R¹⁴,
Z⁴ représente un groupe alkyle en C₂-C₂₀ non substitué ou un groupe allyle en C₁-C₂₀ portant un ou plusieurs substituants, identiques ou différents, halogène, alkylthio, alkylsulfinyle, alkylsulfonyle, alcoxy, alkylamino, dialkylamino, halogénoalkylthio, halogénoalkylsulfinyle, halogénoalkylsulfonyle, halogénoalcoxy, halogéno-alkylamino, halogéno-dialkylamino, -SiR¹²R¹³R¹⁴ et/ou cycloalkyle en C₃-C₆, le fragment cycloalkyle pouvant pour sa part porter éventuellement un ou plusieurs substituants, identiques ou différents, halogène et/ou alkyle en C₁-C₄,
Z⁵ représente un groupe alcényle en C₂-C₂₀ ou alcynyle en C₂-C₂₀ portant éventuellement chacun un ou plusieurs substituants, identiques ou différents, halogène, alkylthio, alkylsulfinyle, alkylsulfonyle, alcoxy, alkylamino, dialkylamino, halogénoalkylthio, halogénoalkylsulfinyle, halogénoalkylsulfonyle, halogénoalcoxy, halogéno-alkylamino, halogéno-dialkylamino, -SiR¹²R¹³R¹⁴ et/ou cycloalkyle en C₃-C₆, le fragment cycloalkyle pouvant pour sa part porter éventuellement un ou plusieurs substituants, identiques ou différents, halogène et/ou allyle en C₁-C₄,
Z⁶ représente un cycle saturé ou insaturé à 3-7 chaînions, éventuellement une ou plusieurs fois substitué, qui contient un atome de silicium en tant que chaînon formant le cycle, Q représentant alors une liaison directe ou un groupe alkylène en C₁-C₄,
R¹⁰ représente un atome d'hydrogène ou un groupe alkyle en C₁-C₄,
R¹¹ représente un atome d'hydxogéne ou un groupe alkyle en C₁-C₄,
m représente 0, 1, 2 où 3,
R¹² et R¹³ représentent, indépendamment l'un de l'autre, un atome d'hydrogène, un groupe alkyle en C₁-C₈, alcoxy en C₁-C₈, alcoxy(C₁-C₄)-alkyle(C₁-C₄), alkyl(C₁-C₄)thio-alkyle(C₁-C₄) ou halogénoalkyle en C₁-C₆,
R¹⁴ représente un atome d'hydrogène, un groupe alkyle en C₁-C₈, alcoxy en C₁-C₈, alcoxy (C₁-C₄)-alkyle(C₁-C₄), alkyl(C₁-C₄)thio-alkyle(C₁-C₄), alcényle en C₂-C₈, alcynyle en C₂-C₈, halogénoalkyle en C₁-C₆, halogénoalcényle en C₂-C₆, halogénoalcynyle en C₂-C₆, cycloalkyle en C₃-C₆, ou un groupe phényle ou phénylalkyle chacun éventuellement substitué,
ou
M-Q-Z représentent ensemble un groupe 1H-2,3-dihydro-indén-4-yle, 1,3-dihydro-2-benzofuran-4-yle ou 1,3-dihydro-2-benzothién-4-yle chacun éventuellement une à trois fois substitué par méthyle,
A représente A1 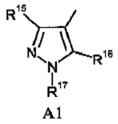
R¹⁵ représente un atome d'hydrogène ou d'halogène, un groupe cyano, nitro, alkyle en C₁-C₄, alcoxy en C₁-C₄, alkyl(C₁-C₄)thio, cycloalkyle en C₃-C₆, halogénoalkyle en C₁-C₄, halogénoalcoxy en C₁-C₄ ou halogénoalkyl(C₁-C₄)thio comportant chacun de 1 à 5 atomes d'halogène, un groupe aminocarbonyle ou aminocarbonyl-alkyle(C₁-C₄),
R¹⁶ représente un atome d'hydrogène ou d'halogène, un groupe cyano, alkyle en C₁-C₄, alcoxy en C₁-C₄ ou alkyl(C₁-C₄)thio,
R¹⁷ représente un atome d'hydrogène, un groupe alkyle en C₁-C₄, hydroxyalkyle(C₁-C₄), alcényle en C₂-C₆, cycloalkyle en C₃-C₆, alkyl(C₁-C₄)thio-alkyle(C₁-C₄), alcoxy(C₁-C₄)-alkyle(C₁-C₄), halogénoalkyle en C₁-C₄, halogénoalkyl(C₁-C₄)thio-alkyle(C₁-C₄), halogénoalcoxy(C₁-C₄)-alkyle(C₁-C₄) comportant chacun 1 à 5 atomes d'halogène, ou un groupe phényle.

2. Carboxamides selon la revendication 1, de formules (I-a), (I-b), (I-e), (I-f) ou (I-d) 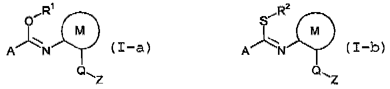 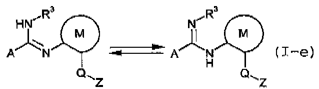 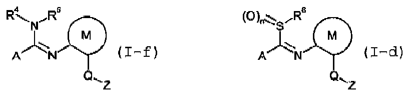 dans lesquelles
R¹, R², R³, R⁴, R⁵, R⁶, M, Q, Z et A ont les significations indiquées dans la revendication 1,
n représente 1 ou 2.

3. Procédé pour la préparation des carboxamides de formule (I) selon la revendication 1, **caractérisé en ce que**
(a) dans le cas où X représente OR¹, on fait réagir des carboxamides de formule (II-a) 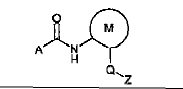 dans laquelle M, Q, Z et A ont les significations indiquées dans la revendication 1,
éventuellement en présence d'une base, avec un agent d'alkylation de formule (III-a)
LG¹-R¹ (III-a)
dans laquelle
R¹ a les significations indiquées dans la revendication 1 et
LG¹ représente un groupe partant,
ou avec un sel de Meerwein de formule (III-d)
(R¹)₃O⁺E⁻ (III-d)
dans laquelle
R¹ a les significations indiquées dans la revendication 1 et
E représente BF₄, SbF₆ ou SbC1₆,
et on obtient des carboxamides de formule (I-a) 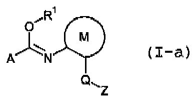 dans laquelle R¹, M, Q, Z et A ont les significations indiquées dans la revendication 1,
(b) dans le cas où X représente SR², SOR⁶ ou SO₂R⁶, dans une première étape on fait réagir des carboxamides de formule (II-a) 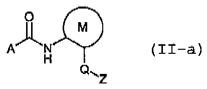 dans laquelle M, Q, Z et A ont les significations indiquées dans la revendication 1,
éventuellement en présence d'un diluant, avec un agent de thionation
et on obtient ainsi des carboxamides de formule (II-b) 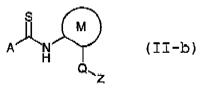 dans laquelle M, Q, Z et A ont les significations indiquées dans la revendication 1,
que, dans une deuxième étape, on fait réagir, éventuellement en présence d'une base, avec un agent d'alkylation de formule (III-b) ou de formule (III-c)
LG²-R² (III-b)
LG⁶-R⁶ (III-c)
formules dans lesquelles
R² et R⁶ ont les significations indiquées dans la revendication 1 et
LG² représente un groupe partant
LG⁶ représente un groupe partant,
et on obtient des carboxamides de formule (I-b) ou de formule (I-c) 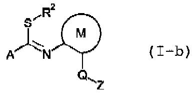 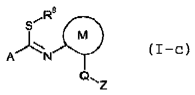 formules dans lesquelles R², R⁶, M, Q, Z et A ont les significations indiquées dans la revendication 1,
et dans une troisième étape on fait ensuite réagir les carboxamides de formule (I-c) avec un oxydant, éventuellement en présence d'un diluant, éventuellement en présence d'un capteur d'acide et éventuellement en présence d'un catalyseur, et on obtient ainsi les carlaoxamides de formule (I-d) 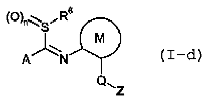 dans laquelle
R⁶, M, Q, Z et A ont les significations indiquées dans la revendication 1 et
n représente 1 ou 2,
(c) dans le cas où X représente NHR³ ou NR⁴R⁵, on fait réagir des carboxamides de formule (II-b) 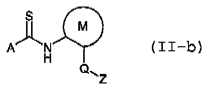 dans laquelle M, Q, Z et A ont les significations indiquées dans la revendication 1,
éventuellement en présence d'une base, avec un agent d' amidation de formule (IV-a) ou de formule (IV-b) 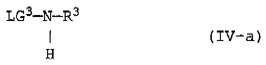 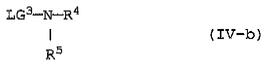 formules dans lesquelles
R³, R⁴ et R⁵ ont les significations indiquées dans la revendication 1 et
LG³ représente un groupe partant,
et on obtient des carboxamides de formule (I-e) ou de formule (I-f) 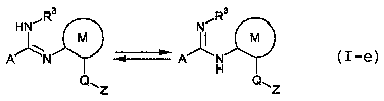 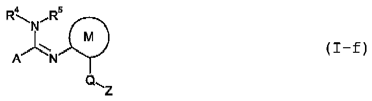 formules dans lesquelles R³, R⁴, R⁵, M, Q, Z et A ont les significations indiquées dans la revendication 1.

4. Composition destinée à la lutte contre des micro-organismes indésirables, **caractérisée par** une teneur en au moins un carboxamide de formule (I) selon la revendication 1, en plus d'excipients er/ou de substances tensioactives.

5. Utilisation des carboxamides de formule (I) selon la revendication 1, pour la lutte contre des micro-organismes indésirables.

6. Procédé pour la lutte contre des micro-organismes indésirables, **caractérisé en ce qu'** on applique sur les micro-organismes et/ou leur habitat des carboxamides de formule (I) selon la revendication 1.

7. Procédé pour la préparation de compositions destinées à la lutte contre des micro-organismes indésirables, **caractérisé en ce qu'** on mélange des carboxamides de formule (I) selon la revendication 1 avec des excipients et/ou des substances tensioactives.
